# EUROPEAN PATENT SPECIFICATION

(11) **EP 3 953 912 B1**
(45) Date of publication and mention of the grant of the patent: **20.11.2024**
(21) Application number: 20787575.8
(22) Date of filing: 01.04.2020
(51) Int. Cl.: G07F 17/00, G07F 11/62

(54) **HANDLING MEDICATION RECEPTACLES BY PHARMACEUTICAL DISPENSING SYSTEM AND METHOD**
HANDHABUNG VON MEDIKAMENTENBEHÄLTERN DURCH PHARMAZEUTIKUMAUSGABESYSTEM UND -VERFAHREN
MANIPULATION DE RÉCEPTACLES DE MÉDICAMENTS PAR UN SYSTÈME DE DISTRIBUTION PHARMACEUTIQUE ET PROCÉDÉ

(30) Priority: 10.04.2019 US 201916379835; 10.04.2019 US 201916379831; 04.06.2019 US 201916430456; 04.09.2019 US 201916559716
(43) Date of publication of application: 16.02.2022
(73) Proprietor: Tech Pharmacy Services, LLC, Fort Lee NJ 07024 (US)
(72) Inventor: EINAV, Omer, 4287500 Kfar-Monash (IL); SHABANOV, Doron, 4486200 Tzur-Yigal (IL); BEN DAVID, Tamir, 6998827 Tel-Aviv (IL); LIVSCHITZ, Eyal, 5440123 Givat Shmuel (IL); MCKINNEY, Thomas A., Boonton, New Jersey 07005 (US); LIBERMAN, Moshe, 5627504 Yehud (IL); SPERO, Anthony Joseph, Queensbury, New York 12804 (US)
(74) Representative: RGTH
(86) International application number: PCT/IB2020/053082
(87) International publication number: WO 2020/208479

(56) References cited:
- EP-B1- 2 457 550
- US-A- 6 036 812
- US-A1- 2017 267 453
- US-A1- 2020 388 100
- US-A1- 2022 101 976
- US-B2- 8 219 243
- US-B2- 8 521 327
- US-B2- 9 908 704

## Description

### RELATED APPLICATIONS

This application claims the benefit of priority under 35 USC §119(e) of U.S. Provisional Patent Application No. 16/430,456 filed on June 4, 2019.

### FIELD AND BACKGROUND OF THE INVENTION

The present invention, in some embodiments thereof, relates to a medication dispensing system and, more particularly, but not exclusively, to handling and positioning of medication receptacles in a medication dispensing system.

US Patent Publication No. 2013/0123977 discloses "systems and methods for managing canisters used to automatically dispense medication. Canisters are configurable via a design process and a build process to accurately dispense a variety of medications. Design profiles are created and stored by a canister management system, and are federated to workstations used to build and fill the canisters, and to workstations used to dispense the medication. Information related to the build process, the fill process, and the dispense process is also federated by the system. The system also enables the transmission of other types of messages between client applications on the workstations and the canister management system. The system is useful to federate data regardless of a structure of a supply chain used to design, build, distribute, and use the canisters".

International Patent Publication No. WO 2018/052160 discloses "a medication dispenser having high space utilization, having a large quantity of medication packages loaded therein, having high medication-dispensing efficiency, and enabling smooth dispensing regardless of the size and type of the medication package. Provided is the medication dispenser comprising: a canister module in which a canister having the medication packages loaded therein is accommodated; and a pickup robot for picking up the medication packages in individual units, wherein the canister includes: L-shaped first and second walls for providing a loading space allowing the medication packages to move therein in the long axis direction of the canister; a guide for moving the first wall toward the second wall so as to adjust a gap with the second wall; a contact plate moving along the loading space, and bringing the medication packages into close contact with each other by pressure; and a spiral spring providing the pressure to the contact plate, having a strip shape, and wound in a coil shape".

US Patent Publication No. 2018/0122177 discloses "storage and distribution system for products in unit doses, including a plurality of housing units, each including a plurality of locations for products in unit doses. The housing units are organized on a vertical plane to produce at least one portion of a picking wall, in which the locations for products in unit doses face selective picking members. A picking unit includes picking members oriented on the picking wall for picking products packaged in unit doses. A collecting unit, arranged on a second side of the picking unit, includes a rack having a plurality of pegs facing towards the first side of the picking unit. The pegs are reached by the picking members so as to pick therefrom or deposit thereon products packaged in unit doses. The plurality of pegs as a whole can collect a smaller number of unit dose products than those that can be stored in the automatic store". Similar medication dispensing systems are described in US Patent publication No. US6036812A and European Patent publication No. EP2457550B1

### SUMMARY OF THE INVENTION

According to an aspect of some embodiments of the present invention there is provided a medication dispensing system, which inserts a probe to pick a medication dosage from a medication container, and dispenses the medication dosage into an opening in a medication receptacle. According to some embodiments, the system includes a medication panel, having a plurality of docking ports for accommodating the medication containers, one or more actuators, a gripper attachable to the probe or including the probe, a receptacle carrier comprising a receptacle mount for holding the receptacle, and movable by the one or more actuators. In some embodiments, the system includes control circuitry, outputting positioning signals to the one or more actuators to move the receptacle carrier, and outputting dosage-manipulation signals to the one or more actuators to move the gripper to pick and manipulate the medication dosage out of the medication container. In some embodiments, a horizontal distance between the opening of the receptacle and the medication dosage is less than 20 cm at least prior to outputting the dosage-manipulation signals.

According to some embodiments, the positioning signals maintain a horizontal distance of less than 20 cm between a projection of the opening of the receptacle and a projection of the medication container on a horizontal plane.

According to some embodiments, the dosage-manipulation signals manipulate the medication dosage in a medication path, between the medication container and the opening of the medication receptacles, having a total horizontal length of less than 20 cm.

According to some embodiments, the system includes a dispensing head, supporting the receptacle carrier to form a single unit that moves the receptacle together with the dispensing head. In some embodiments, the gripper is rotatably coupled to the dispensing head. In some embodiments, the dispensing head comprises a head housing, and a movable based platform rotatably coupled to the head housing. In some embodiments, the gripper is coupled to the base platform. In some embodiments, the gripper is linearly moveable in respect to the head housing.

According to some embodiments, the manipulation signals include rotating the gripper, between picking a medication dosage out of the medication container and positioning the medication dosage vertically above the opening of the receptacle.

According to some embodiments, the receptacles are medication envelopes, having an open state in which an upper side of the envelope is open for receiving medication dosage.

According to some embodiments, the system includes an envelope opener module having a manipulator, configured to open the envelope by coupling the manipulator to a face of the envelope.

According to some embodiments, the one or more actuators move the receptacle carrier vertically between the medication containers.

According to some embodiments, at said horizontal distance, there is an overlap between the projection of the opening of the receptacle and the projection of the medication dosage on a horizontal plane, so that the medication dosage falls into the opening when released from said probe. According to some embodiments, the one or more of the actuators or the dispensing head, actuate the receptacle carrier to move horizontally to be in a constant horizontal distance between the opening of the receptacle and the medication dosage.

According to some embodiments, the receptacle carrier is configured to hold more than one receptacle.

According to some embodiments, the receptacle is configured to accommodate one or more medication dosages.

According to some embodiments, the dispensing system comprises one or more output ports, and the receptacle carrier is movable to deliver the receptacle to the one or more output ports.

According to some embodiments, the dispensing system comprises a plurality of dispensing heads.

According to some embodiments, the dispensing system comprises a plurality of receptacle carriers.

According to some embodiments, the receptacle carrier is de-coupled of the dispensing head after dispensing the medication dosage in a receptacle.

According to some embodiments, there is an overlap between the projection of the opening of the receptacle and the projection of the medication container on a horizontal plane, between or during outputting the positioning signals and the dosage manipulation signals.

According to some embodiments, the receptacle carrier is coupled to the dispensing head at least prior to outputting dosage manipulation signals. In some embodiments, the receptacle carrier is coupled to the dispensing head prior to outputting approximating signals. In some embodiments, the receptacle carrier is coupled to the dispensing head after outputting approximating signals.

According to some embodiments, the control circuitry is configured to receive one or more parameters of the medication dosage, to process one or more velocity profiles defined according to the one or more parameters of the medication dosage. In some embodiment, the control circuitry outputs manipulation signals having one or more velocity profiles.

According to an aspect of some embodiments of the present invention there is provided a method for dispensing medications in receptacles, using a dispensing system, having a gripper module for pick a medication dosage from a medication container, and dispenses the medication dosage into an opening in a medication receptacle. According to some embodiments, the method includes extracting the medication dosage out of the medication container by the gripper module or by coupling the gripper module to a probe inserted in the medication container, positioning a receptacle by a receptacle carrier in a horizontal distance of less than 20 cm between the medication dosage and the opening of the receptacle, at least prior to the extracting, and dispensing the medication dosage in the receptacle.

According to some embodiments, the method includes locating the medication dosage to be vertically above the opening in the medication receptacle. According to some embodiments, the positioning comprises moving the receptacle carrier in respect to the gripper.

According to some embodiments, the method includes opening the receptacle prior to the dispensing. According to some embodiments, the method includes opening the receptacle between the positioning and the dispensing. According to some embodiments, the time between the extracting and the dispensing is less than 1 sec.

According to some embodiments, the method includes rotating the gripper module to align with a probe inserted within the medication container prior to the extracting.

According to some embodiments, the dispensing system includes a dispensing head supporting the gripper module, and the method includes coupling the receptacle carrier and the dispensing head prior to the extracting.

According to some embodiments, the positioning is prior to the extracting. According to some embodiments, the positioning includes maintaining an overlap between the projection of the opening of the receptacle and the projection of the medication container on a horizontal plane.

According to some embodiments, the method includes approximating the gripping module to the medication container.

According to some embodiments, the method includes closing the receptacle after the dispensing.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As will be appreciated by one skilled in the art, some embodiments of the present invention may be embodied as a system, method or computer program product. Accordingly, some embodiments of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, some embodiments of the present invention may take the form of a computer program product embodied in one or more computer readable medium(s) having computer readable program code embodied thereon. Implementation of the method and/or system of some embodiments of the invention can involve performing and/or completing selected tasks manually, automatically, or a combination thereof. Moreover, according to actual instrumentation and equipment of some embodiments of the method and/or system of the invention, several selected tasks could be implemented by hardware, by software or by firmware and/or by a combination thereof, e.g., using an operating system.

For example, hardware for performing selected tasks according to some embodiments of the invention could be implemented as a chip or a circuit. As software, selected tasks according to some embodiments of the invention could be implemented as a plurality of software instructions being executed by a computer using any suitable operating system. In an exemplary embodiment of the invention, one or more tasks according to some exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized for some embodiments of the invention. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some embodiments of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some embodiments of the present invention may be described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Some of the methods described herein are generally designed only for use by a computer, and may not be feasible or practical for performing purely manually, by a human expert. A human expert who wanted to manually perform similar tasks, such as positioning medication receptacles in respect to a medication picking probe and dispensing medication dosage in proximity to a medication container in a dispensing system, might be expected to use completely different methods, e.g., making use of expert knowledge and/or the pattern recognition capabilities of the human brain, which would be vastly more efficient than manually going through the steps of the methods described herein.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Figure 1 is a simplified illustration of a block diagram of a pharmaceutical dispensing system, according to some embodiments of the invention;
Figure 2 is a simplified illustration of a block diagram of a pharmaceutical dispensing system, according to some embodiments of the invention;
Figures 3A-3D are simplified flow charts illustrating dispensing process, according to some embodiments of the invention;
Figure 4A is a simplified illustration of a perspective view of a medication container panel, according to some embodiments of the invention;
Figure 4B is a simplified illustration of a perspective view of a medication container panel, according to some embodiments of the invention;
Figure 5 is a simplified illustration of a block diagram of a dispensing head, according to some embodiments of the invention;
Figures 6A-6C are simplified illustrations of a side view of a dispensing system, according to some embodiments of the invention;
Figure 7A is a simplified illustration of a side view and a front view of a dispensing system, according to some embodiments of the invention;
Figure 7B is a simplified illustration of a side view and a top view of a dispensing system, according to some embodiments of the invention;
Figure 7C is a simplified illustration of a side view and a top view of a dispensing system, according to some embodiments of the invention;
Figures 8A and 8B are simplified illustrations of a perspective view of an envelope supply unit, according to some embodiments of the invention;
Figures 9A and 9B are simplified illustrations of a perspective view and a top view of a portion of an envelope carrier, according to some embodiments of the invention;
Figures 9C and 9E are simplified illustrations of perspective views of a portion of an envelope carrier, according to some embodiments of the invention;
Figure 9D is a simplified illustration of a side view of a portion of an envelope carrier, according to some embodiments of the invention; and
Figure 9F is a simplified illustration of a top view of a portion of an envelope carrier, according to some embodiments of the invention; and
Figures 10A, 10B and 10C are simplified flow charts, illustrating exemplified workflows of operating a pharmaceutical dispensing system, according to some embodiments of the invention.

### DESCRIPTION OF SPECIFIC EMBODIMENTS OF THE INVENTION

The present invention, in some embodiments thereof, relates to a medication dispensing system and, more particularly, but not exclusively, to handling and positioning of medication receptacles in a medication dispensing system.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Overview

A broad aspect of some embodiments of the invention relates to a medication dispensing system for extracting medication dosage out of medication containers and depositing the medication dosage in receptacles provided to the system.

According to some embodiments, the dispensing system has an interior arrangement and interactions configured to affect operational parameters of the medication dispensing system. In some embodiments, the dispensing system comprises a control circuitry and actuators electrically connected to the control circuitry that defines and controls operational parameters of the medication dispensing system. In some embodiments, the operational parameters include minimizing the movements of the medication dosage. In some embodiments, the operational parameters include moving the medication dosage in short movements after being extracted from medication container and prior to being deposited into a receptacle.

An aspect of some embodiments of the invention relates to reducing accidental depositing of medication dosage outside medication receptacles in a medication dispensing system configured to extract one or more medication dosages out of medication containers and deposit the medication dosage in medication receptacles.

According to some embodiments, the dispensing system is mechanically structured to perform short movements of medication dosage to reduce an accidental loosing of the extracted medication dosage prior to the depositing of the medication dosage inside the medication receptacle.

According to some embodiments the medication dispensing system comprises a gripper for picking the medication dosage, and the dispensing system keeps the movements of the medication dosage to be minimal by positioning the medication receptacles in proximity to the gripper. In some embodiments, dispensing system positions the medication receptacles in proximity to the gripper during the extracting of the medication dosage out of the container and the depositing of the medication dosage into the medication receptacles.

According to some embodiments, the system includes a control circuitry that controls the movements of the medication dosage. In some embodiments, the control circuitry outputs signals to one or more actuators that manipulate the medication dosage over a path, referred hereinafter as "a medication path", between the medication container and the receptacle, said medication path having a total horizontal length (defined as the projection of the medication path on a horizontal plane) of less than 20 cm.

A potential advantage in keeping the receptacles in proximity to the gripper is reducing the length and time that the medication dosage travels outside the medication container.

An aspect of some embodiments of the invention relates to a medication dispensing system, having a safe destination for a medication dosage during a dispensing process.

According to some embodiments, the dispensing system comprises a receptacle carrier for carrying the medication receptacles and keeping the receptacles in proximity to the medication dosage. In some embodiments, the medication receptacles comprise an opening acting as a funnel for receiving the medication dosage. In some embodiments, the horizontal distance between the medication dosage and the opening of the receptacle is kept to be less than 20 cm. In some embodiments, the horizontal distance between the medication dosage and the opening of the receptacle is defined as the maximal distance between the projection of the medication dosage on a horizontal plane and the projection of the opening of the receptacle on a horizontal plane.

In some embodiments, the receptacle carrier holds and positions the opening of the medication receptacle in a horizontal distance of less than 20 cm between the extraction of the medication dosage and the depositing of the medication dosage. In some embodiments, the receptacle carrier holds and positions the opening of the medication receptacle vertically below the medication dosage between the extraction of the medication dosage and the depositing of the medication dosage.

According to some embodiments the medication dispensing system comprises a gripper for picking medication dosage. In some embodiments, the medication dispensing system comprises one or more actuators for moving the gripper. In some embodiments, the actuators position the gripper in proximity to a medication container prior to extracting a medication dosage by the gripper. In some embodiments, the dispensing system couples the gripper to a probe, said probe inserted in the medication containers for picking the medication dosage out of the medication container.

According to some embodiments, the dispensing system includes a dispensing head and the gripper is coupled to the dispensing head. In some embodiments, the gripper is movably coupled to the dispensing head. In some embodiments, the gripper is rotatable coupled to the dispensing head. In some embodiments, the actuators move the dispensing head to position the gripper in respect to the medication container. In some embodiments, the actuators move the dispensing head to position the gripper in respect to the receptacle.

In some embodiments, the receptacle carrier is coupled to the dispensing head, so that the receptacle is moving together with the dispensing head. In some embodiments, the receptacle carrier is coupled to the dispensing head prior to picking a medication dosage and at least until the depositing of the medication into the receptacle. In some embodiments, the dispensing head and the receptacle carrier are a single unit.

In some embodiments, the medication receptacles are medication envelopes.

In some embodiments, the probe is used for picking medication dosage out of the containers, and for depositing the medication. In some embodiments, the probe is coupled to the medication containers and extracting medication dosage is by grabbing the probe out of the container by the gripper. In some embodiments, depositing of the medication dosage is by dropping it from the gripper or probe to the opening of the receptacle positioned below the gripper.

According to some embodiments, there is a horizontal overlap between a projection of the opening of the receptacle and a projection of the medication container on a horizontal plane. In some embodiments, the receptacle is positioned below the probe in a horizontal distance of less than 20 cm between picking of the probe and depositing of the medication. In some embodiments, the time between the picking of the probe out of the container and the depositing of the medication dosage is shorter than 10 sec.

A potential advantage is reducing the time is the potential avoidance of accidentally losing the medication dosage from the probe prior to depositing the medication dosage. Another potential advantage is minimizing the time the medication container is left open when probe is removed from the container.

An aspect of some embodiments of the invention relates to a medication dispensing system, which extracts a medication dosage out of medication containers and dispenses the medication dosage via an opening formed in medication envelopes during the dispensing process.

According to some embodiments, the dispensing system comprises an envelope opener module configured to open the medication envelopes at a specific period during the dispensing process. In some embodiments, the envelope opener module opens the envelope in the period between the extracting of the medication dosage and the depositing of the medication dosage in the envelope.

According to some embodiments, multiple medication dosages can be dispensed within a single envelope. In some embodiments, when the envelope is not open, it is kept closed to avoid contamination of medication within the envelope.

According to some embodiments, the system includes envelope carrier, configured to hold and to open the medication envelope. In some embodiments, the system has a control circuitry and one or more actuators, and the control circuitry outputs signals to the one or more actuators to actuate the carrier to open the envelope. In some embodiments, the envelope opener module is coupled to the envelope carrier.

An aspect of some embodiments of the invention relates to a medication dispensing system, having a control circuitry outputting signals to actuate one or more actuators to manipulate a medication dosage, between picking of the medication dosage out of a medication container and depositing the dosage in a medication receptacle.

According to some embodiments, the control circuitry processes the timing of the signals and the value of the signal in accordance to one or more medication parameters. In some embodiments, the signals include one or more velocity profiles. In some embodiments, the signals include one or more acceleration values. In some embodiments, the signals include the length and the direction of the movements. Some examples of the medication parameters are weight, type, shape, and cost. In some embodiments, control circuitry includes storage for storing historical data such as medication parameters, velocity profiles, and success/failure rate of dispensing medication dosage having velocity profile selected according to the medication parameters.

### Medication Dispensing System

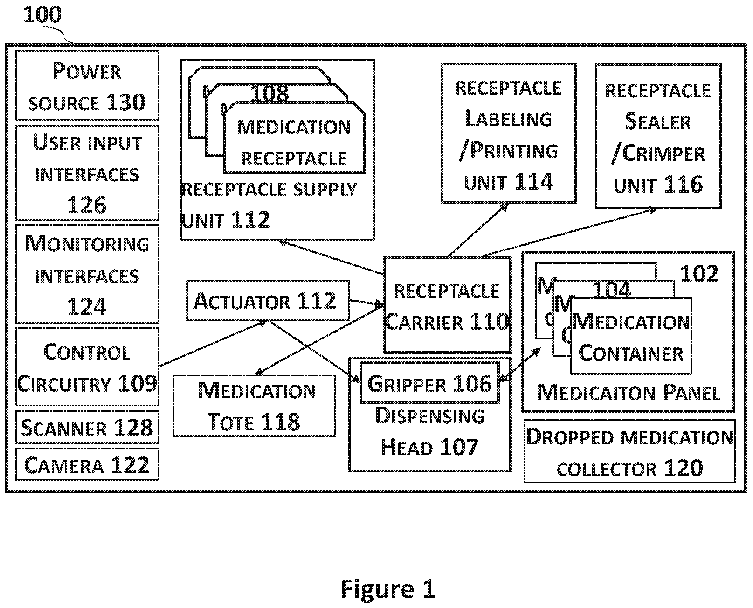

Referring now to Fig. 1, which is a simplified illustration of a block diagram of a pharmaceutical dispensing system, according to some embodiments of the invention.

As shown in Fig. 1, medication dispensing system **100** includes one or more medication panels **102** for accommodating a supply of medications. According to some embodiments, medication is stored in medication containers **104.** In some embodiments, medication panels **102** have a plurality of docking ports for coupling medication containers **104** to panels **102.**

Dispensing system **100** has a medication gripper **106** configured to receive a medication dosage from medication containers **104** and to hold the medication dosage until disposing the medication dosage in a medication receptacle **108.** According to some embodiments, gripper **106** is configured to move next to panel **102** and approximate a container **104** for receiving a medication dosage. In some embodiments, dispensing system **100,** has a plurality of grippers **106.** In some embodiments, medication gripper **106** is configured to dispense medication dosage in a plurality of medication receptacles **108.**

According to some embodiments, dispensing system **100** dispenses the medication dosage by dropping the medication dosage into medication receptacles **108** optionally positioned under medication gripper **106.** In some embodiments, dispensing system **100** moves gripper **106** above medication receptacles **108** to allow dropping the medication dosage into medication receptacles **108.** In some embodiments, dispensing system **100** moves medication receptacles **108** under gripper **106** to allow dropping the medication dosage into medication receptacles **108.** In some embodiments, disposing of the medication dosage is by manipulating the medication dosage into receptacles **108** positioned in proximity to medication gripper **106.**

As shown in Fig. 1, dispensing system **100** includes one or more receptacle carriers **110** for manipulating one or more medication receptacles **108** having an opening for receiving medication dosage. In some embodiments, receptacle carrier **110** manipulates the medication receptacles **108** to follow the position of medication gripper **106.** In some embodiments, receptacle carrier **110** manipulates the medication receptacles **108** to have their opening located vertically beneath medication gripper **106** in a horizontal distance of less than 30 cm. In some embodiments, receptacle carrier **110** manipulates the medication receptacles **108** to have their opening located vertically beneath medication gripper **106** in a horizontal distance of less than 20 cm. In some embodiments, receptacle carrier **110** manipulates the medication receptacles **108** to have their opening located vertically beneath medication gripper **106** in a horizontal distance of less than 10 cm. In some embodiments, there is an overlap between a projection of the opening of receptacle **108** and a projection of the medication dosage on a horizontal plane, so that the medication dosage falls into the opening of receptacle **108** when released from gripper **106.** In some embodiments, there is an overlap between the projection of the opening of receptacle **108** and the projection of container **104** on a horizontal plane.

A potential advantage in moving medication receptacles **108** in proximity to medication gripper **106** is reducing the length and time that the gripper **106** travels with a medication dosage for dropping the dosage into receptacle **108.** Another potential advantage is reducing the risk of losing medication dosage between receiving the dosage out of container **104** and dropping into receptacle 108. For example, an overlap can potentially increase the chances of receiving a medication dosage by receptacle 108 if medication dosage falls or it is released from gripper **106.**

In some embodiments, gripper **106** does not move horizontally towards receptacle **108** after receiving the medication dosage. A potential advantage of reducing the horizontal movements is reducing the medication preparation time to increase the medication packaging rate. Another potential advantage is reducing the risk of losing medication dosage from gripper **106.**

According to some embodiments, dispensing system **100** includes a control circuitry **109** configured to output actuation signals to actuate gripper **106** and receptacle carrier **110.** In some embodiments, system **100** actuates gripper **106** and receptacle carrier **110** using one or more actuators **111** by delivering actuating signals from the control circuitry **109.**

According to some embodiments, the control circuitry **109** outputs approximation signals to actuators **111** to move gripper **106** to approximate medication container 104. In some embodiments, the control circuitry **109** outputs dosage manipulation signals to actuators **111** to move gripper **106** to manipulate medication dosage out of medication container **104.** In some embodiments, the control circuitry **109** outputs positioning signals to actuators **111** to move gripper **106** to position the opening of receptacle **104** in a horizontal distance of less than 30 cm from the medication dosage. In some embodiments, the control circuitry **109** outputs positioning signals between or during the approximating signals and the dosage manipulation signals.

According to some embodiments, multiple medication dosages can be dispensed within a single medication receptacle **108.** A potential advantage in dispensing multiple medication dosages in a single medication receptacle **108** is reducing the travel of gripper **106** and/or carrier **110** to collect receptacles and/or deliver packaged receptacles. Another potential advantage is reducing the number of receptacles packaged per patient.

According to some embodiments, receptacle carrier **110** is movable independently of gripper **106.** In some embodiments, dispensing system **100** has one or more carrier actuators **111** that move receptacle carrier **110.** In some embodiments, carrier actuators move receptacle carrier **110** linearly. In some embodiments, carrier actuators move carrier **110** parallel to panel **102.** In some embodiments, moving receptacle carrier **110** is synchronous with moving of gripper **106.**

According to some embodiments, dispensing system **100** includes a dispensing head **107** and gripper **106** is coupled to the dispensing head **107.** In some embodiments, gripper **106** is movably coupled to dispensing head **107.** In some embodiments, gripper **106** is rotatable coupled to dispensing head **107.** In some embodiments, actuators **110** move the dispensing head **107** to position the gripper **106** in respect to a medication container **104.** In some embodiments, actuators **110** move the dispensing head **107** to position the gripper **106** in respect to a receptacle **108.**

According to some embodiments, dispensing system **100** is mechanically configured to move the receptacle carrier **110** together with the dispensing head **107.** In some embodiments, receptacle carrier **110** is configured to be attached to head **107** prior to dispensing medication. In some embodiments, receptacle carrier **110** is configured to be attached to head **107** prior to approximating container **104.** In some embodiments, receptacle carrier **110** is configured to be attached to head **107** prior to picking medication dosage by gripper **106.** In some embodiments, receptacle carrier **110** is configured to be attached to head **107** prior to dispensing medication dosage. In some embodiments, attaching carrier **110** to head **107** is by moving head **107** towards receptacle carrier **110.** In some embodiments, attaching carrier **110** to head **107** is by moving receptacle carrier **110** towards head **107.** In some embodiments, carrier **110** and head **107** are part of one unit.

According to some embodiments, receptacle carrier **110** is configured to carry one or more medication receptacles **108.** In some embodiments, receptacle carrier **110** receives and holds medication receptacles **108** prior to dispensing medication dosage. In some embodiments, receptacle carrier **110** receives and holds medication receptacles **108** prior to extracting medication dosage from medication containers **104.**

In some embodiments, control circuitry **109** outputs positioning signals to actuators **111** to move receptacle carrier **110** to position the opening of receptacle **104** in a horizontal distance of less than 20 cm from the medication dosage. In some embodiments, the horizontal distance is less than 10 cm from the medication dosage. In some embodiments, the horizontal distance is less than 5 cm from the medication dosage. In some embodiments, the horizontal distance between the medication dosage and the opening of the receptacle **104** is defined as the maximal distance between the projection of the medication dosage on a horizontal plane and the projection of the opening of the receptacle **104** on a horizontal plane.

In some embodiments, control circuitry **109** outputs positioning signals to actuators **111** to move receptacle carrier **110** between medication containers **104.** In some embodiments, control circuitry **109** outputs positioning signals between or during the approximating signals and the dosage manipulation signals. In some embodiments, moving receptacle carrier **110** between medication containers **104** is defined as a movement of a receptacle **108** from being positioned in proximity to one container **104** to being positioned in proximity to another container **104.** In some embodiments, the movement of a receptacle **108** between containers is horizontal. In some embodiments, the movement of a receptacle **108** between containers **104** is vertical. In some embodiments, the movement of a receptacle **108** between containers **104** is without increasing the distance of receptacle **108** from panel **102.** According to some embodiments, medication receptacles **108** are medication envelopes. In some embodiments, medication receptacles **108** have 3D geometry, such as rectangular box, cylindrical, conical, etc. In some embodiments, medication receptacles **108** are rigid like plastic canister. In some embodiments, medication receptacles **108** are non-rigid like plastic, nylon bag, and paper.

According to some embodiments, dispensing system **100** includes one or more receptacle supply units **112** for storing receptacles **108.** In some embodiments, carrier **110** is configured to acquire receptacles 108 from receptacle supply unit **112.** In some embodiments, supply unit **112** is loaded with receptacles **108** of different sizes. In some embodiments, the dispensing system **100** has a plurality of supply unit **112** fitted to accommodate receptacles **108** of different types as described elsewhere herein.

According to some embodiments, dispensing system **100** has a labeling or printing unit **114** for labeling medication receptacles **108** with information related to medications disposed in receptacle **108,** e.g. patient information, medication dosage information, time, etc. In some embodiments, printing unit **114** is configured to print on a surface of receptacle **108.** In some embodiments, labeling medication receptacles **108** by placing a sticker on a surface of receptacles **108.**

According to some embodiments, dispensing system **100** has a receptacle sealer **116.** In some embodiments, when receptacles **108** are envelopes, sealer **116** can be a crimping device. In some embodiments, sealer **116** is a device configured for closing receptacles **108** by a lid/cover.

According to some embodiments, dispensing system **100** has one or more output ports for accommodating medication receptacles **108** after having medication dosage. According to some embodiments, one or more of the output ports are in the form of medication totes **118** for accommodating medication receptacles **108** after having medication dosage. In some embodiments, one or more of the output ports are PRN outputs for providing medication not through medication totes **118.**

In some embodiments, the dispensing system **100** comprises a power source **130** configured to supply the necessary energy to the parts of the system **100.** In some embodiments, the power source **130** allows the system **100** to operate without the need of external power sources.

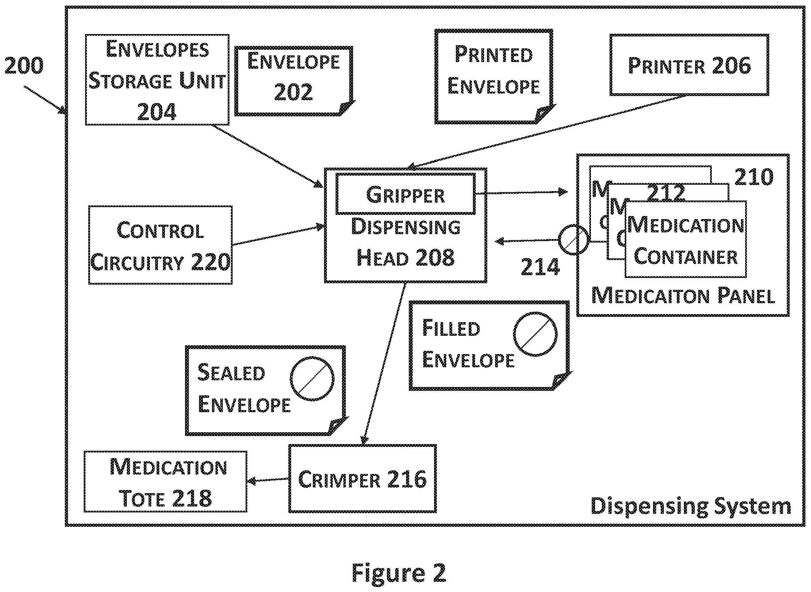

Turning to Fig. 2, which is a simplified block diagram of modules of the dispensing system that participate in handling medication envelopes configured to receive one or more medications, according to some embodiments of the invention.

As shown in Fig. 2, dispensing system **200** comprises one or more envelope storage units **204** for storing medication envelopes **202.**

According to some embodiments, system **200** comprises a label printer **206** configured to print on envelope **202.** In some embodiments, printer **206,** prints on envelope **202** information related to medication dosages disposed therein.

According to some embodiments, system **200** comprises a dispensing head **208** configured to receive and hold envelopes **202** during the dispensing operation. In some embodiments, dispensing head **208** includes an envelope carrier to receive and hold envelopes **202.** In some embodiments, dispensing head **208** receives and hold envelopes **202** by coupling head **208** and envelope carrier.

According to some embodiments, the dispensing head **208** is configured for picking a medication dosage **214** from a medication container **212** located within a medication containers panel **210.** In some embodiments, dispensing head **208** includes a gripper to pick medication dosage **214** from a medication container **212.** In some embodiments, one or more envelopes 202 are coupled to dispensing head **208** prior to picking medication dosage **214.** In some embodiments, the envelopes **202** are coupled to a lower portion of the dispensing head **208** when disposing medication dosage **214** in envelope **202,** such as medication dosage **214** are dispensed by dropping medication dosage **214** from a higher portion of head **208** into envelope **202** located at the lower portion. In some embodiments, envelope **202** is positioned under the gripper prior to dropping medication dosage **214** from the gripper into envelope **202.**

According to some embodiments, envelope **202** has an open state in which the volume of envelope **202** is expanded to allow inserting dosage **214** into the envelope. In some embodiments, head **208** is configured to modify the state of envelope **202** to an open state. In some embodiments, an envelope carrier coupled to head **208** sets the state of envelope **202.** In some embodiments, opening of envelope **202** is after envelope **202** is coupled to head **208.** In some embodiments, head **208** opens envelope **202** prior to disposing medication dosage **214.** In some embodiments, at least 90% of the projection of the receptacle at an open state on a horizontal plane is a funnel for medication dosage, and when a medication dosage is dropped into that projection, the medication dosage will be funneled into the receptacle.

According to some embodiments, dispensing system **200** has a control circuitry **220** that controls the movements and operation of dispensing head **208.** In some embodiments, control circuitry **220** controls the setting the state of envelope **202.** In some embodiments, control circuitry **220** actuates head **208** to set envelope **202** to an open state. In some embodiments, control circuitry **220** actuates head **208** to couple envelope **202** to head **208.** In some embodiments, control circuitry **220** actuates head **208** to pick a medication dosage **214** from a medication container **212** by outputting dosage manipulation signals. In some embodiments, control circuitry **220** actuates head **208** to dispose medication dosage **214** in envelope **202,** by outputting dispensing signals.

According to some embodiments, system **200** comprises a crimper **216,** which receives and seals envelope **202** after being filled with medication dosage **214.** In some embodiments, envelope **202** has a closed state in which envelope **202** is flat, having its volume in minimal state. In some embodiments, holding envelope **202** in an open state is terminated prior to receiving envelope **202** by crimper **216.**

According to some embodiments, system **200** comprises one or more medication totes **218,** to receive and store the sealed envelopes **202.** In some embodiments, facility personnel unload envelopes **202** from medication totes **218** in order to distribute the medications packaged in envelopes **202.**

### Preparation process of medications to be dispensed

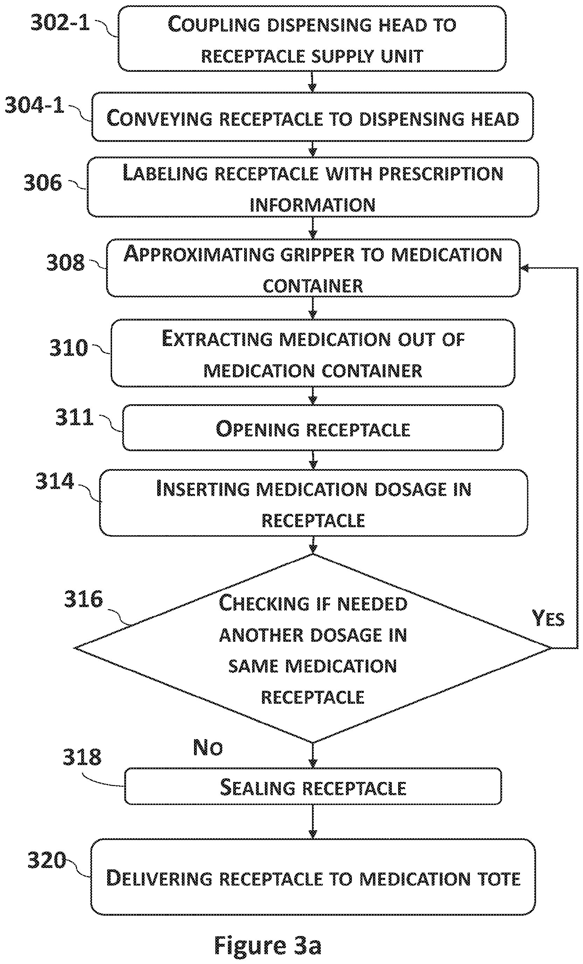

Referring now to Figs. 3A-3D, which are simplified flow charts illustrating the preparation process of medications to be dispensed, according to some embodiments of the invention. Some of the differences between the preparation processes described in Figs. 3A-3D are: the ways the receptacles are positioned in proximity to the dispensing gripper, the order some of the actions, and optional actions such as inserting multiple dosages in a receptacle that can be added to any one of the preparation processes.

According to some embodiments, the preparation process of medications to be dispensed can be divided into the following categories of activities:
Pre-preparation activities, such as: providing a medication receptacle, and positioning the receptacle in proximity to the dispensing gripper.

Preparation activities, such as: extracting medication dosage, and inserting medication in the receptacle. In some embodiments, the insertion steps can be repeated. For example, when multiple dosages are inserted in a single or multiple receptacles.

Post-preparation activities, such as: sealing the receptacles, and placing the receptacles in a collection unit/medication tote.

### Pre-preparation activities

### Coupling a medication receptacle to a dispensing head -

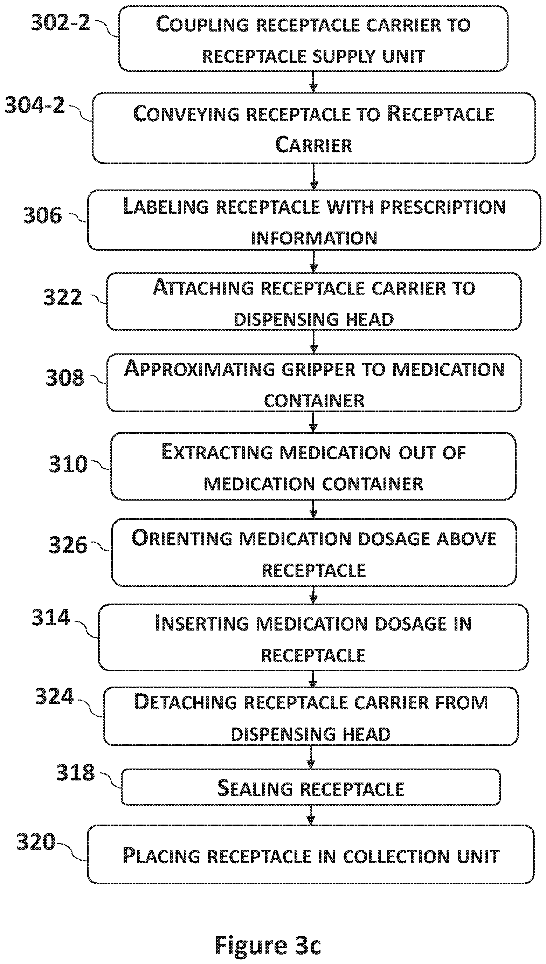

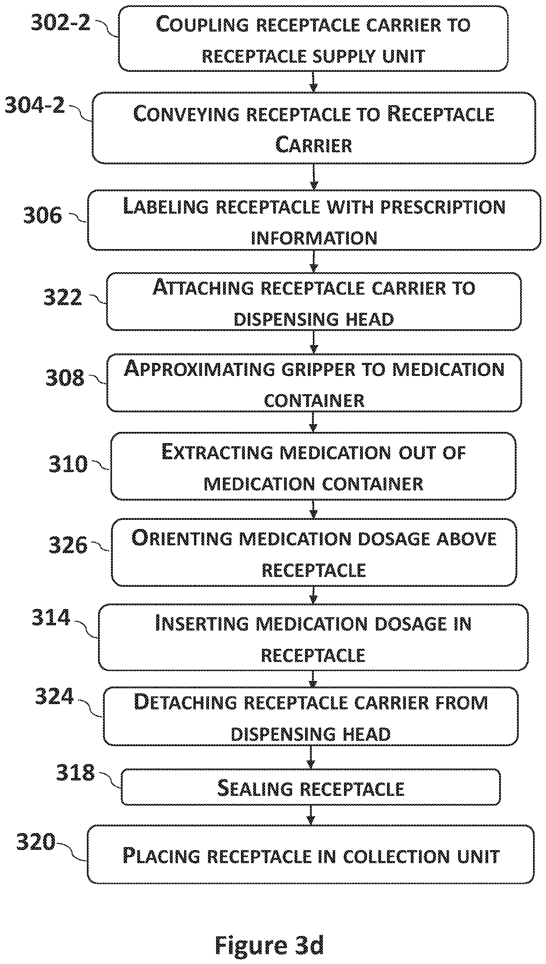

According to some embodiments, for example as show in in Figs. 3A, 3C, and 3D, the medication receptacle (e.g. **108)** is coupled to a dispensing head (e.g. **107)** having a gripper (e.g. **106)** prior to the preparation of the envelopes containing the medication dosage. A potential advantage of coupling the medication receptacles to the dispensing head prior to the preparation, is reducing the travel of the gripper during the preparation process.

As shown in Fig. 3A, according to some embodiments, providing a medication receptacles includes the following steps:
Coupling **302-1** the dispensing head (e.g. **107)** to a receptacle supply unit (e.g. **112);** and
Conveying **304-1** a receptacle (e.g. **108)** from receptacle supply unit (e.g. **112)** to the dispensing head.

As shown in Figs. 3C and 3D, according to some embodiments, providing a medication receptacles is by attaching a receptacle carrier **(110)** to the dispensing head (107) and includes the following steps:
Coupling **302-2** the receptacle carrier to receptacle supply unit for receiving a receptacle;
Conveying **304-2** the receptacle to receptacle carrier; and
Attaching **322** the receptacle carrier to the dispensing head.

### Positioning the gripper next to medication container -

As shown in Figs. 3A-3D, the dispensing process includes approximating **308** the gripper to a medication container (such as **104)** within medication containers panel (such as **102).**

According to some embodiments, approximating is by a linear movement of the gripper. In some embodiments, the linear movement is in a vertical direction. In some embodiments, the linear movement is in one or more horizontal directions. In some embodiments, the linear movement is a combination of horizontal and vertical movements. In some embodiments, the linear movements include movements which are angular to the medication container.

According to some embodiments, approximating is by control circuitry (e.g. **109),** outputting approximation signals to one or more actuators (e.g. **111)** to move the gripper to approximate the medication container.

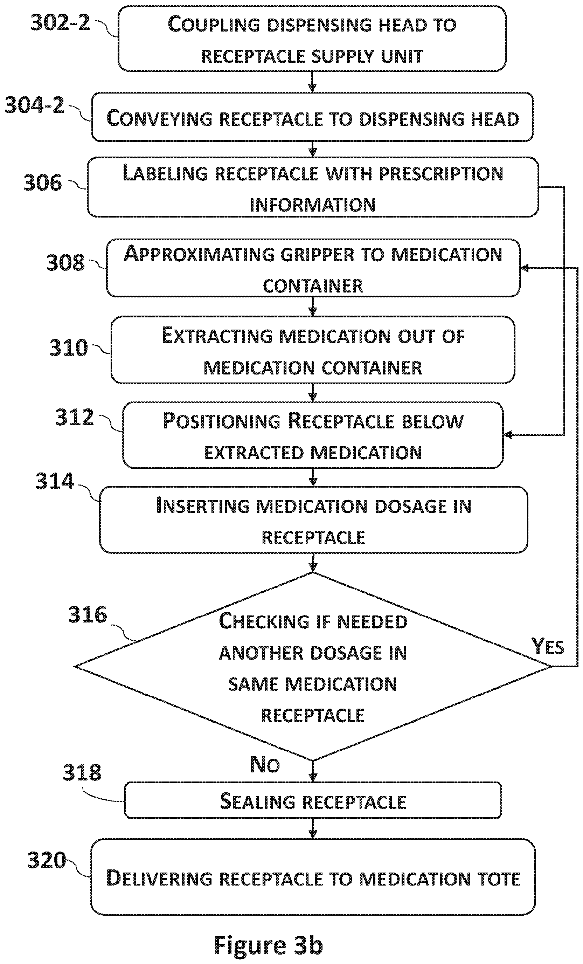

According to some embodiments, for example such as shown in Fig. 3B, one or more of the receptacle preparation steps, such as coupling **302-2,** conveying **304-2,** and labeling **306,** can be performed in parallel to one or more activities performed by the dispensing head or gripper, such as approximating **308** and extracting **310.** A potential advantage in performing receptacle preparation step(s) in parallel to dispensing head or gripper activities is increasing dispensing rate.

### Preparation activities -

### Receiving and holding medication dosage

As shown in Figs. 3A-3D, after medication gripper is positioned next to a medication container that contains a targeted medication, the gripper picks and holds a medication dosage. As shown in Figs. 3A, 3C, and 3D, according to some embodiments, the medication receptacle is coupled to the dispensing head prior to the step of extracting a medication dosage.

According to some embodiments, extracting the medication is by extracting **310** the medication dosage out of the medication container. In some embodiments, extracting **310** is by providing suction through an extraction probe. In some embodiments, extracting is by gripping a medication dosage. In some embodiments, extracting includes lifting an extraction probe out of the medication container.

According to some embodiments, extracting **310** includes outputting dosage manipulation signals by control circuitry to one or more actuators to move one or more of the dispensing head and the gripper to manipulate the medication dosage out of the medication container. In some embodiments, the signals include one or more velocity profiles. In some embodiments, the signals include one or more acceleration profiles. In some embodiments, the signals include the length and the direction of the movements. In some embodiments, outputting the dosage manipulation signals is preceded by receiving one or more parameters of the medication dosage, and processing one or more velocity profiles according to the one or more parameters of the medication dosage. Some examples of the medication parameters are: weight, type, shape, and cost. In some embodiments, the processing of the velocity profiles includes processing of historical data such as medication parameters, velocity profiles, and success/failure rate of dispensing medication dosage having velocity profile selected according to medication parameters.

As shown in Fig. 3C, the preparation process includes orienting **326** the medication dosage to be located above the medication receptacle prior to inserting it into the receptacle. In some embodiments, orienting **326** include orienting the extraction probe. In some embodiments, the distance between the dispensing head and the medication container does not change during orienting **326.** In some embodiments, there is no linear movement of the gripper in a horizontal direction away of the container between extracting and inserting.

As shown in Fig. 3B, the preparation process includes positioning **312** a receptacle below the extracted medication dosage. In some embodiments, positioning **312** applies when receptacle is not earlier coupled to dispensing head prior to extracting medication dosage. In some embodiments, positioning **312** applies when the dispensing system includes a receptacle carrier configured to move independently of the inserting without coupling the receptacle to the dispensing head prior to extracting medication dosage. In some embodiments, positioning **312** is of the opening of the receptacle below the extracted medication dosage.

According to some embodiments, positioning **312** include outputting positioning signals by the control circuitry to one or more actuators to move one or more of: the dispensing head, the gripper, and the receptacle carrier, to position the opening of the receptacle in a horizontal distance of less than 20 cm from the medication dosage between or during the approximating **308** and extracting **310.** In some embodiments, the horizontal distance is less than 10 cm. In some embodiments, the horizontal distance is less than 5 cm. In some embodiments, the horizontal distance is less than 50 cm.

According to some embodiments, for example, when receptacle is an envelope (e.g. **202),** the preparation process includes opening **311** receptacle to an open state. In some embodiments, opening **311** is for expanding the volume of a receptacle envelope to allow inserting medication dosage. In some embodiments, for example as shown in Fig. 3A, opening **311** is between extracting **310** and inserting **314.** In some embodiments, opening **311** is after positioning **312.** Opening **311** is exemplified in Fig. 3A, however, it can apply to the flows described in any one of Figs. 3B-3D, or other embodiments, not described in Figs. 3A-3D.

As shown in Figs. 3A-3D, the preparation process includes inserting **314** the medication dosage in the medication receptacle (e.g. **108).**

In some embodiments inserting **326** is by dropping medication dosage directly from the medication container into the receptacle through a port in the container.

In some embodiments, when medication is held by the medication gripper using suction, inserting is performed by reducing the suction and dropping dosage into the receptacle.

According to some embodiments, for example as shown in Figs. 3A and 3B, inserting **314** is following by the optional step of checking **316** if additional dosage is required to be inserted in the same receptacle. In some embodiments, checking 316 is an optional step in other flow options of the preparation process, such as these shown in Figs. 3C and 3D.

### Post-preparation activities

According to some embodiments, having a probe picked out of a medication container, the post-preparation activities include returning the probe to the medication container. In some embodiments, returning of the probe includes re-orienting and inserting the probe into the medication container. In some embodiments the distance between the dispensing head and the medication container does not change during re-orienting and inserting. According to some embodiments, there is no linear movement of the medication gripper between approximating 308 to a medication container and inserting the probe into the medication container. In some embodiments, there is no linear movement of the medication gripper in a vertical direction between approximating **308** and inserting. In some embodiments, there is no linear movement of the medication gripper in a horizontal direction between approximating **308** and inserting.

According to some embodiments, a receptacle having a medication dosage is sealed and delivered to a receptacle collection zone. Some of the post-preparation activities include:
Sealing **318** the medication receptacle after being filled with medication dosage. According to some embodiments, sealing **318** include covering the receptacle. In some embodiments, covering is by a lid.

In some embodiments, the receptacle is a medication envelope (e.g. **202)** and sealing **318** is by crimping the envelope. In some embodiments, post-preparation activities include closing the envelope to be flat with minimal volume. In some embodiments, closing the envelope is by terminating a force which holds the envelope in an open state is terminated. In some embodiments, closing the envelope is prior to receiving the envelope by the crimper.

Placing **320** the medication receptacles filled with a medication dosage in the collection unit/medication tote. In some embodiments, placing **320** is followed by dispensing the medication receptacles to patients.

According to some embodiments, the preparation process includes labeling **306** the receptacle with prescription information. Labeling **306** can be a pre-preparation step, a preparation steps, or a post-preparation step. In some embodiments, for example as shown in Fig. 3C, labeling **306** is performed prior to attaching **322** to the dispensing head. In some embodiments, for example as shown in Fig. 3D, labeling **306** is after inserting **314.** In some embodiments, labeling 306 is after sealing **318.** In some embodiments, labeling **306** is prior to conveying **304.**

### Medication containers panel

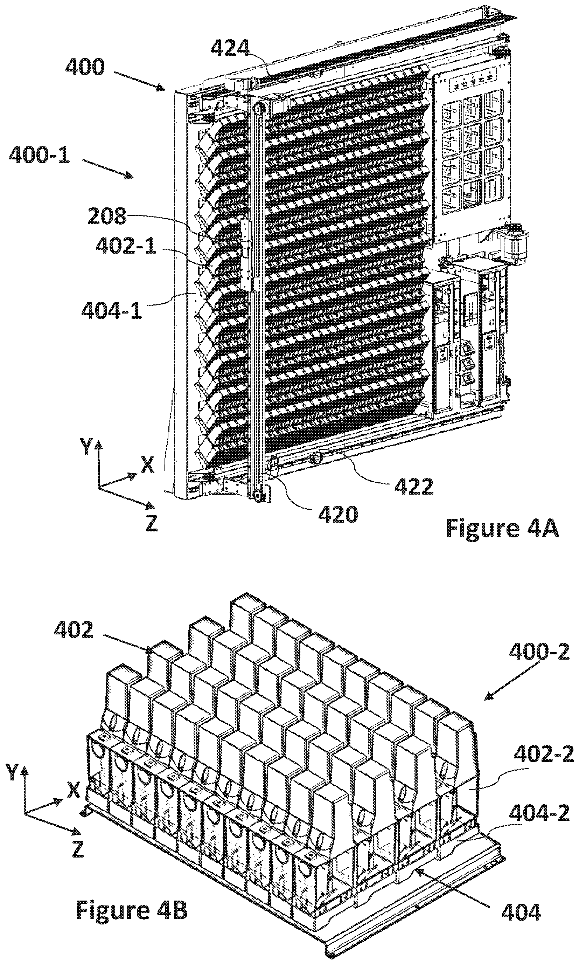

Referring now to Figs. 4A and 4B, which are simplified illustrations of a perspective view of medication containers panels, according to some embodiments of the invention.

As shown in Figs. 4A and 4B, according to some embodiments, medication containers panel **400** accommodates a plurality of medication containers **402.** In some embodiments, panel **400** has a plurality of docking ports **404** for coupling medication containers **402.**

As shown in Fig. 4A, the medication containers **402** are arranged vertically (in direction Y) within a container panel **400-1.** In some embodiments, panel **400-1** is vertically flat. In some embodiments, containers **402** are slanted on panel **400-1.** In some embodiments, containers **402** are slanted by shaping docking ports **404-1** to couple containers to be in a slanted orientation.

In some embodiments, the panel is slanted. In some embodiments, the panel is arcuate. In some embodiments (not shown), the panel is cylindrical.

A potential advantage of having a medication panel extending vertically is reducing the horizontal size of the panel. In some embodiments, reducing the horizontal size of the panel reduces the horizontal size of the dispensing system.

As shown in Fig. 4B, according to some embodiments, the medication dispensing system has medication container panel **400-2,** configured to accommodate a plurality of medication containers **402,** arranged horizontally within container panel **400-2.** In some embodiments, panel **400-2** is horizontally flat. In some embodiments, panel **400-2** is circular or arcuate about a vertical axis (Y).

A potential advantage of having a medication panel extending horizontally is reducing the vertical size of the panel. In some embodiments, reducing the vertical size of the panel reduces the vertical size of the dispensing system. In some embodiments, disposing the medication containers **402** in a horizontal arrangement reduces the horizontal size of the system.

According to some embodiments, as shown in Fig. 4A, a dispensing head (e.g. **107/208/600)** described elsewhere herein is moveable on a vertical rail **420** moveable mounted on medication panel **400,** and the dispensing head is configured to move vertically on vertical rail **420.** In some embodiments, the dispensing head is configured to move horizontally by moving vertical rail **420** on or more horizontal rails **422/424** provided at panel **400.** According to some embodiments (not shown), the dispensing head is moveable on a horizontal rail coupled to the medication panel, and the linear movement of the head is on the horizontal rail. In some embodiments, a vertical movement of the dispensing head is by moving the horizontal rail on or more vertical rails provided at the medication panel.

### Dispensing head with receptacle carrier

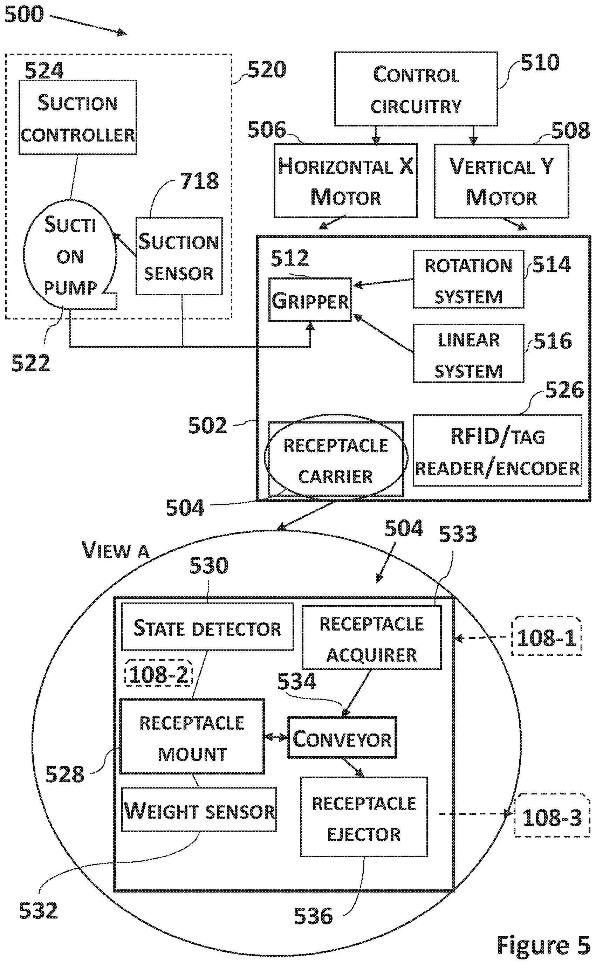

Referring now to Fig. 5, which is a simplified illustration of a block diagram of a dispensing head, according to some embodiments of the invention.

Dispensing head **500** is configured to grab medication from medication containers accommodating medication. According to some embodiments, dispensing head **500** is configured to move in one or more directions to approximate medication containers **402** and receive a medication dosage. According to some embodiments, e.g. when the layout of the medication panel is vertical (e.g. **400-1),** approximating a medication container is by moving dispensing head **500** in at least a vertical direction (e.g. direction Y in Fig. 4A). In some embodiments, e.g. when the layout of the medication panel is horizontal (e.g. **400-2),** moving dispensing head **500** is at least in a horizontal direction (e.g. directions X and Z in Fig. 4B).

As shown in Fig. 5, dispensing head **500** includes one or more base platforms **502** for holding modules of head **500.** According to some embodiments, head **500** includes a gripper **512** for picking medication dosage out of a container. In some embodiments, gripper **512** is a probe gripper, configured to pick a probe which holds medication dosage. In some embodiments, gripper is configured to apply suction in a probe to pick and hold medication by the probe.

According to some embodiments, dispensing head **500** includes a receptacle carrier **504** for coupling one or more medication receptacles such as **108** to head **500.**

According to some embodiments, dispensing head **500** is moveable by one or more motors. In some embodiments, motor **506** moves head **500** in horizontal direction X. In some embodiments, motor **508** moves head **500** in vertical direction Y. In some embodiments, one of motors **506** or **508** moves head **500** in horizontal direction Z. In some embodiments, one or more of motors **506/508** are actuated by control circuitry **510.** In some embodiments, control circuitry **510** is coupled to dispensing head **500.** In some embodiments, for example as shown in Fig. 5, control circuitry **510** is located outside head **500.** In some embodiments, motor **506** is coupled to head **500.** In some embodiments, for example as shown in Fig. 5, motor **506** is disposed outside head **500** and motor motion is transferred to head **500.** In some embodiments, motor **508** is coupled to head **500.** In some embodiments, for example as shown in Fig. 5, motor **508** is disposed outside head **500** and motor motion is transferred to head **500.** In some embodiments, one or more of motors **506/508** are step motors. In some embodiments, one or more of motors **506/508** are servo motors.

According to some embodiments, the dispensing system includes a suction system **520** connected to head **500,** for attaching medication dosage to an extraction probe as described elsewhere herein. In some embodiments, suction is provided by suction system **520** to gripper **512** for applying suction through the probe. In some embodiments, suction system **520** includes a suction pump **522.** In some embodiments, suction system **520** includes a suction controller **524** connected to suction pump **520** for controlling the suction provided to gripper **512.** In some embodiments, suction controller **524** is disposed within head **500.** In some embodiments, determining the required suction power is according to the characteristic and parameters of the medication dosage (e.g. type, shape, weight, etc.). In some embodiments, suction system **520** includes a suction sensor for measuring suction power provided to gripper **512.** In some embodiments, the suction sensor is connected to gripper **512.** In some embodiments suction power is controlled by one or more suction valves coupled to dispensing head **500.**

According to some embodiments, control circuitry **510** controls the movement speed of head **500** according to the characteristic and parameters of the medication dosage (e.g. type, shape, weight, etc.). A potential advantage of controlling the speed of head **500** and/or suction power is potentially reducing the possibility of losing of a medication dosage coupled to dispensing head **500** after being extracted out of the container. Another potential advantage of controlling the suction power is reducing of failures in extracting medication dosage by the dispensing head **500.**

According to some embodiments, gripper **512** is linearly moveable in respect to platform **502.** In some embodiments, gripper **512** is configured to grab a probe inserted in a medication container by approximating gripper **512** to the probe for grabbing the probe away of the container by a linear motion. In some embodiments, gripper **512** is configured to return the probe to the container by a linear motion towards of the container. In some embodiments, gripper **512** is actuated to move linearly by a linear system **516** coupled to platform **502.** In some embodiments, moving gripper **512** in linear motion is without moving platform **502** in respect to the medications panel.

According to some embodiments, a picked probe is rotated by gripper **512** to be positioned above a medication receptacle for inserting the extracted medication dosage into the medication receptacle. In some embodiments, gripper **512** is rotationally coupled to platform **502.** In some embodiments, rotating of gripper **512** is by rotation system **514** coupled to platform **502.** In some embodiments, head **500** is configured to move the gripper **512** in a rotational motion without moving platform **502** in respect to the medication panel. In some embodiments, there is no linear movement of gripper **512** in a horizontal direction away of the container between the extracting and the inserting of medication dosage. In some embodiments, the rotation is in the range of 10 to 85 deg. In some embodiments, the rotation is in the range of 25 to 75 deg. In some embodiments, the rotation is in the range of 30 to 60 deg.

In some embodiments, control circuitry **510** outputs positioning signals to actuators **514/516** to move gripper **512** to position the opening of the receptacle in a horizontal distance of less than 10 cm from the medication dosage. In some embodiments, control circuitry **510** outputs positioning signals between or during outputting approximating signals and outputting dosage manipulation signals.

A potential advantage in limiting the motion of the probe by head **500** is that the time the medication container is left open when probe is out of the container minimized. In some embodiments, the time between grabbing of the probe out of the container by gripper **512** and inserting the medication dosage in the medication receptacle is shorter than 10 sec. In some embodiments, the time between grabbing of the probe out of the container by gripper **512** and inserting the medication dosage in the medication receptacle is shorter than 4 sec. In some embodiments, the time between grabbing of the probe out of the container by gripper **512** and inserting the medication dosage in the medication receptacle is shorter than 1 sec. Another potential advantage is reducing accidental loosing of the medication dosage from the probe prior to inserting the medication dosage inside the medication receptacle.

According to some embodiments, head **500** is configured to communicate with the medication container via a chip or RFID tag mounted at the container, or by using barcode at the container. In some embodiments, the RFID/chip is used to transmit operational parameters of the container. Some examples of information that can be communicated via RFID/chip are: identifying medication within container, counting medication dosages, receiving status details, preparation status, etc. In some embodiments, head **500** is updating information encoded on the container. For example: updating medication dosage remaining within container after extracting dosage by head. In some embodiments, head **500** has a RFID/tag reader and/or encoder **524.** In some embodiments, read/encoder **526** is coupled to platform **502.** In some embodiments, read/encoder **526** is coupled to gripper **512.**

According to some embodiments, reader/encoder is movable to enable approximating and detracting to/from the medication container. In some embodiments, read/encoder **526** is configured to move together with gripper **512.** For example, for approximating RFID tag/Chip of container, while picking a probe by gripper **512.** In some embodiments, reader/encoder **526** is configured to move independently of gripper **512.** In some embodiments, reader/encoder **526** is configured to rotate with gripper **512,** and to move linearly independently of gripper **512.** A potential advantage in moving reader/encoder **526** is that the head **500** is communicating with medication containers, without further moving the head **500.** This can help minimizing travel for the head **500** and reducing preparation process time. A communication of head **500** with container can also help avoiding an initiation of medication extraction in case reader **526** detects some unexpected input from a chip of the container, e.g. out of medication. Such exception can proceed to moving head **500** automatically to another container, without proceeding a faulty dispensing process. This can increase usability, by reducing fault handling by an operator. This can also reduce idling of the dispensing system.

Turning to **View A** in Fig. 5, which is a simplified block diagram illustration of receptacle carrier 504 according to some embodiments of the present invention.

According to some embodiments, receptacle carrier **504** includes receptacle mount **528,** which is configured to hold one or more receptacles (e.g. **108)** when inserting medication dosage into the receptacle.

According to some embodiments, the receptacles are attached to head **500** by coupling the receptacle to a receptacle mount **528.** In some embodiment, coupling is by suction power applied to the receptacle by mount **528.** In some embodiments, suction system **512** is connected receptacle carrier **504** to provide the suction to mount **528.** In some embodiments, suction system **512** connected to carrier **504** is different than suction system connected to gripper **512.**

According to some embodiments, carrier **504** includes a state detector **530** for determining if the receptacle held by mount **528** is in an open state or a closed state. In some embodiments, dispensing operation is performed only when state is open. In some embodiments, detector **530** is connected to control circuitry **510** to control dispensing of medication based on receptacle open/closed state. A potential advantage of detecting the open/close state of the receptacle is reducing the risk of missing medication dosage within receptacle. Another potential advantage is reducing waist of medication dosages dispensed into closed receptacles.

According to some embodiments, the weight of the receptacle increases when dispensing medication dosage. In some embodiments, a weight sensor **532** is used for measuring the weight of the receptacle. In some embodiments, suction provided to receptacle mount **528** is determined according to weight measured by sensor **532.** In some embodiments, suction controller **516** increases the suction power when weight measurement increases. In some embodiments, measuring of the weight is used to verifying medication insertion within receptacle.

According to some embodiments, receptacle carrier **504** acquires receptacles from a receptacle supply unit (such as **112).** In some embodiments, carrier **504** includes a receptacle acquirer **533,** which acquires receptacle from the supply unit. In some embodiments, head **500** is positioned next to engage acquirer **533** with the supply unit.

According to some embodiments, transferring receptacles outside carrier **504** is required one or more time during a preparation process. In some embodiments, carrier **504** includes a receptacle ejector **536,** configured for ejecting receptacle from carrier **504.** In some embodiments, receptacles are ejected from head **500** to a medication output/collection unit, such as medication tote (such as **118),** after inserting medication into receptacle.

In some embodiment, acquiring by receptacle acquirer **533** and transferring receptacles outside carrier **504** by ejector **536** is repeated more than once during a single preparation process. For example: acquiring receptacle from receptacle supply unit (e.g. **112)** and then transferring receptacle to a labeling or printing unit (such as **114)** and back to head carrier **504.** Another example: transferring receptacle to and from sealing/crimping unit (such as **116)** prior to disposing receptacle in an output/collection unit, such as medication tote (e.g. **118).**

According to some embodiments, receptacle carrier **504** includes a conveyor **534** for transporting receptacles from acquirer **533** to mount **528,** e.g. prior to inserting medication within the receptacle. In some embodiments, transporting receptacles from mount **528** to ejector **536,** e.g. after inserting medication is by conveyor **534.** In some embodiments, transporting to ejector **536** is to discard unfilled receptacles, e.g. when an error occurs during the preparation process.

In some embodiments, dispensing head **500** is configured to hold multiple receptacles by having a plurality of receptacle carriers **504.** In some embodiments, dispensing head **500** is configured to hold multiple receptacles by having a plurality of receptacle mounts **528** within receptacle carriers 504.

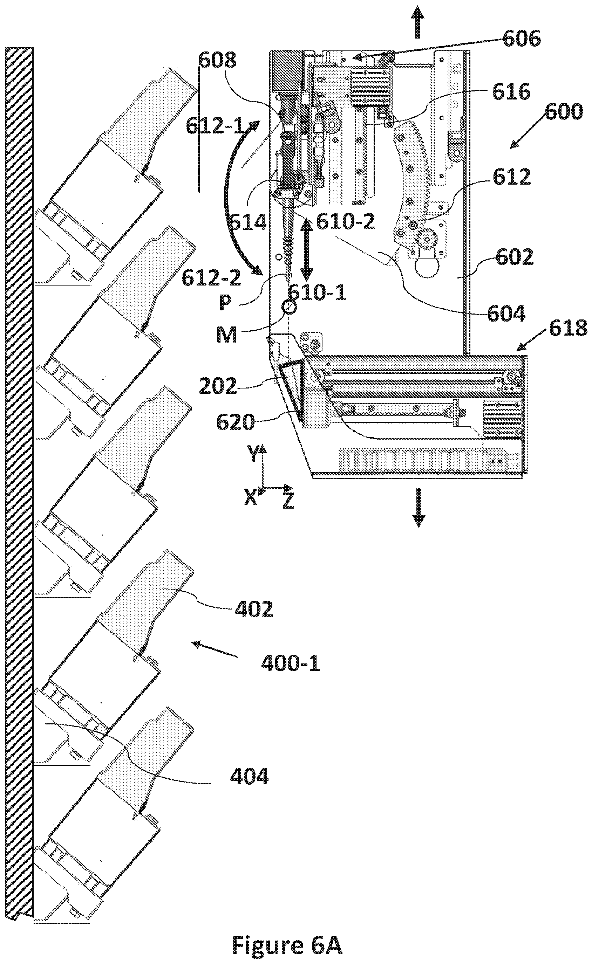

Referring now to Figs. 6A-6C, which are simplified illustrations of a side view of a dispensing head, according to some embodiments of the invention.

As shown in Fig. 6A, dispensing head **600** is configured to move across a vertical panel **400-1** and approximate containers **402** coupled to panel **400-1.** Head **600** is configured to move between containers **402** by a linear movement in a vertical direction Y. In some embodiments, the linear movement is a combination of horizontal movements in direction X and vertical movement in direction Y across panel **400-1.**

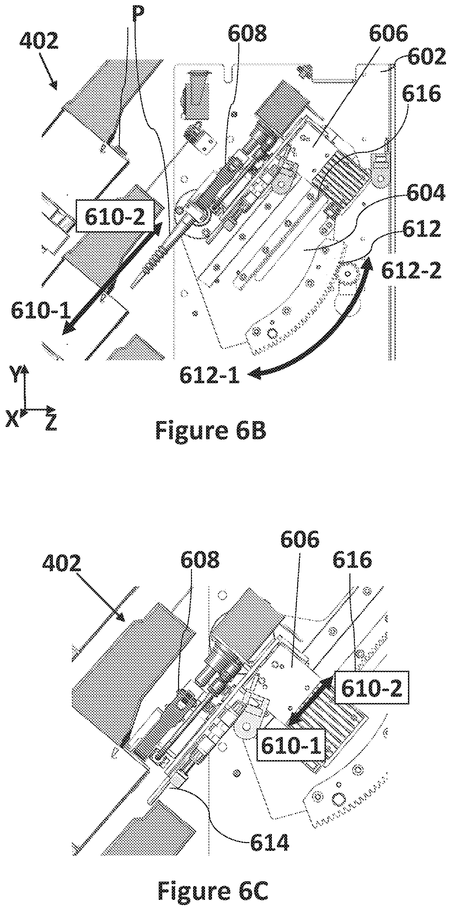

As shown in Figs. 6A and 6B, dispensing head **600** includes a head housing **602.** In some embodiments, head **600** has a movable platform **604,** rotatably coupled to housing **602.**

According to some embodiments, head **600** includes a gripper module **606,** coupled to platform **604,** and a gripper **608** coupled to gripper module **606** and configured for picking a probe **P** coupled to a medication container **402.**

As shown in Figs. 6A to 6C, in some embodiments, gripper module **606** is linearly moveable in respect to housing **602** in a proximal direction **610-1** and a distal direction **610-2.** In some embodiments, gripper module **606** is configured to actuate gripper **608** for grabbing a probe **P** from the container by approximating gripper **608** to the probe **P** in a proximal direction **610-1,** and grabbing the probe away of the container by a distal linear motion in direction **610-2.** In some embodiments, returning of probe **P** to the container is by actuating gripper **608** by gripper module **606** in a proximal linear motion towards of the container in direction **610-1.** In some embodiments, moving gripper **608** in proximal direction **610-1** and distal direction **610-2** is without moving housing **602** in respect to the medications panel (such as **400-1).** In some embodiments, proximal direction **610-1** and distal direction **610-2** are vertical in direction Y.

According to some embodiments, rotating gripper **608** in respect to the medications panel is without rotating housing **602** and without moving head **600.** In some embodiments, for example as shown in Figs. 6A and 6B, head **600** includes a gear mechanism **612,** interconnecting platform **604** and housing **602.** In some embodiments, rotating of platform **604** by gear **612,** rotates gripper **608** in directions **612-1** (shown in Fig. 6B) and **612-2** (shown in Fig. 6A). In some embodiments rotational directions **612-1** and **612-2** are about axis X which is perpendicular to axes Y and Z.

According to some embodiments, as described elsewhere herein, head is configured to update information encoded on the container. As shown in Figs. 6A and 6C, head **600** includes a RFID/tag reader and/or encoder **614,** coupled to gripper module **606.** In some embodiments, gripper module **606** is configured to actuate reader/encoder **614** in a proximal direction **610-1** and a distal **610-2** direction, to enable approximating and detracting to/from an RFID tag/Chip coupled to a medication container **402.**

In some embodiments, reader/encoder **614** is movable in respect to housing **602.** In some embodiments, for example as in Figs. 6A to 6C, reader/encoder **614** is configured to move together with gripper **608,** for example, for approximating RFID tag/Chip of container, while picking a probe by gripper **608.** In some other embodiments, reader/encoder **614** is configured to move independently of gripper **608.**

In some embodiments, head **600** includes a linear gear mechanism **616,** interconnecting gear module **606** and housing **602.** In some embodiments, moving of gear module **606** by gear **616,** moves gripper **608** in directions **610-1** (shown in Fig. 6C) and **610-2** (shown in Figs. 6A and 6B). In some embodiments, linear gear mechanism **616,** interconnects gear module **606** and platform **604.** In some embodiments, connecting gear **616** to platforms **604,** enables actuating of gripper **608** and/or reader **614** in both linear and rotation motion in respect to housing **602.**

According to some embodiments, dispensing head **600** includes envelope carrier **618** for coupling one or more medication envelopes (such as **202)** to head **600.** In some embodiments, for example as shown in Fig. 6A, envelope carrier **618** is coupled to housing **602,** below gripper **608.** According to some embodiments, envelope carrier **618** includes envelope mount **620,** which is configured to hold an envelope (e.g. **202)** when dispensing medication dosage into the envelope. As shown in Fig. 6A, envelope mount **620,** is holding envelope **202** vertically under gripper **608.** In some embodiments, when a probe **P** is used to hold the medication dosage, envelope mount **620,** is holding envelope **202** vertically under the tip of the probe P, such as a medication disposed at the tip of the probe is dispensed by dropping the medication **M** from the probe **P** into the open envelope.

In some embodiment, holding the envelope is by suction power applied to a surface of the envelope by mount **620.** In some embodiments, a suction system (such as **520)** is connected to envelope carrier 618 to provide suction to a suction port disposed in mount **620.**

### Dispensing head detached of Receptacle carrier

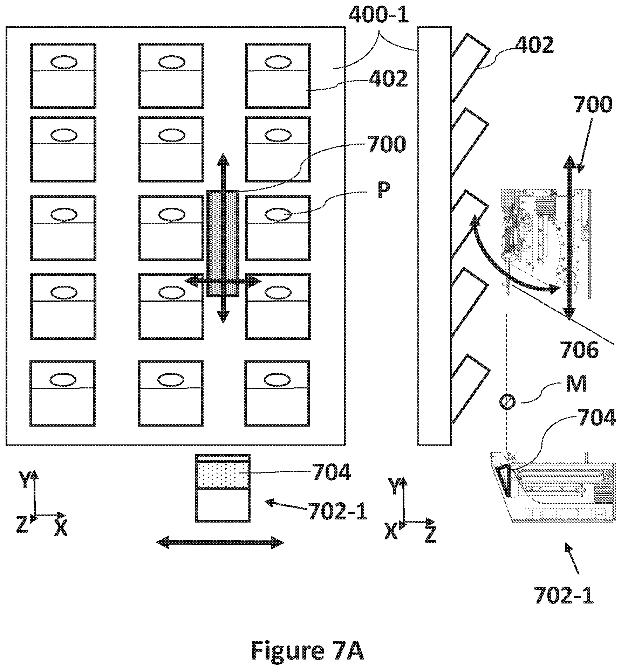

Turning to Figs. 7A to 7C, which are simplified illustrations of side views of dispensing heads and medication container panels, according to some embodiments of the invention.

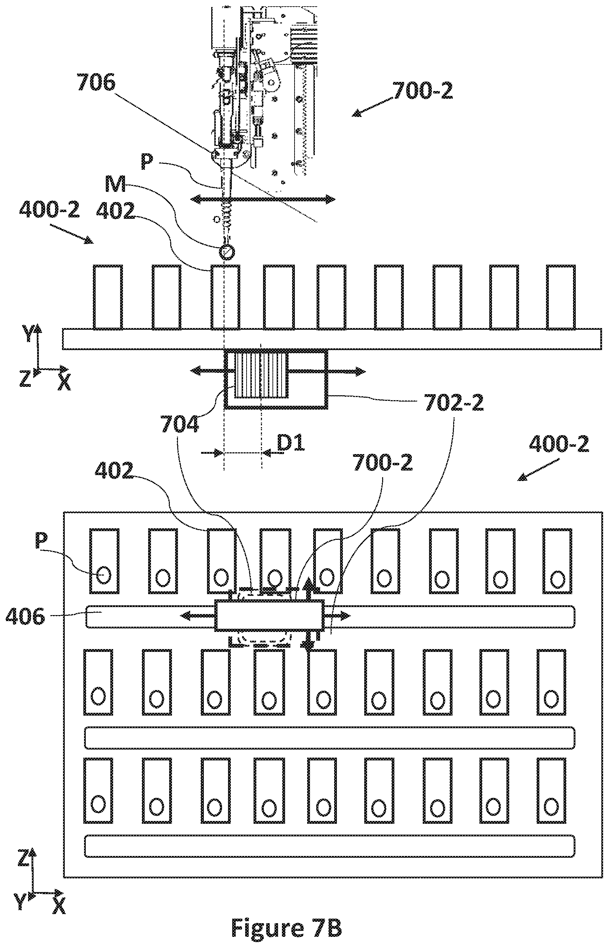

Fig. 7A shows a size view and a front view of an embodiment of a vertical containers panel **400-1,** and Fig. 7B shows a size view and a top view of an embodiment of a horizontal containers panel **400-2.** In both embodiments, carrier **702-1/2** is decoupled of dispensing head **700-1/2.**

According to some embodiments, one or more carrier actuators move carrier **702-1/2** horizontally. In some embodiments, for example as shown in Figs. 7A and 7B, carrier **702-1/2** moves receptacle **704** horizontally below medications panel **400-1/2.**

In some embodiments, moving horizontally, is to maintain a horizontal distance D1 between head **700** and an opening of receptacle **704** within a pre-define range. In some embodiments, distance D1 is shorter than 50 cm. In some embodiments, distance D1 is shorter than 10 cm. In some embodiments, distance D1 is shorter than 20 cm. In some embodiments, distance D1 is shorter than 5 cm. In some embodiments, receptacle **704** is vertically below dispensing head **700.** In some embodiments, for example as shown in Fig. 7B, distance D1 is measured between gripper **706** coupled to dispensing head **700,** and an opening of receptacle **704.** In some embodiments, for example as shown in Fig. 7B, distance D1 is measured between the location of holding a medication dosage M by gripper **706** and an opening of receptacle **704.** In some embodiments, the location of holding a medication dosage **M** is a tip of probe **P** (as discussed elsewhere herein).

In some embodiments, for example as shown in Fig. 7B, the distance **D1** is controlled by moving both head **700-2** and carrier **702-2** in a horizontal direction (e.g. direction X).

In some embodiments, dispensing system includes a control circuitry having a carrier controller for actuating the carrier **702-1/2.** In some embodiments, the control circuitry is actuating carrier **702-1/2** according to an optimization algorithm, which determines the shorter travel of the dispensing head after receiving a medication dosage. In some embodiments, control circuitry controls the speed of moving carrier **702-1/2** according to a speed optimization algorithm. In some embodiments, as described elsewhere herein, speed is controlled to reduce loosing of medication dosage prior to dispensing into receptacle.

According to some embodiments, moving carrier **702-1/2** to position carrier **702-1/2** at a horizontal distance from dispensing head is prior to approximating container **402.** In some embodiments, moving carrier **702-1/2** to position carrier **702-1/2** at a pre-defined horizontal distance from dispensing head is after dispensing head **700-1/2** receives a medication dosage and prior to dispensing it into the receptacle **704.**

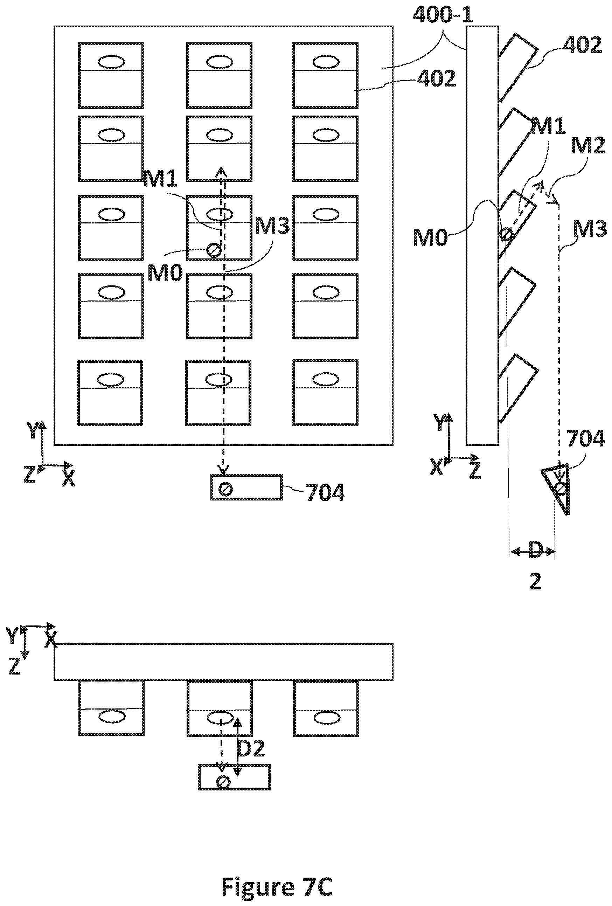

As shown in Fig. 7A, carrier **702-1,** is holding envelope receptacle **704** vertically under dispensing head **700,** such as a medication is dispensed by head **700** by dropping the medication **M** into the open receptacle **704.** Fig. 7C, illustrates an exemplified path **M0-M3** of medication dosage **M,** from being disposed **M1** in container **402,** extracted **M1** out of container **402,** positioned **M2** away of container **402,** and dropped **M3** into a funnel defined by the open envelope **704** located vertically underneath medication dosage **M** after being extracted from container **402.**

In some embodiments, for example as shown in Fig. 7B, horizontal panel **400-2** includes slots **406,** which allow to dispense a medication dosage **M** from head **700-2** located at one side of panel **400-2** (e.g. top of panel) to receptacle **704,** located at an opposite side of panel **400-2** e.g. bottom of panel).

According to some embodiments, carrier **702-1/2** is independent of head **700** and is configured to be attached to head **700** prior to dispensing medication. In some embodiments, a vertical carrier actuator, moves carrier **702** vertically to couple carrier **702** to dispensing head **700** during the dispensing process (as described elsewhere herein).

### Receptacles envelopes

As described elsewhere herein, receptacles are in some embodiments of the invention in the form of medications envelopes.

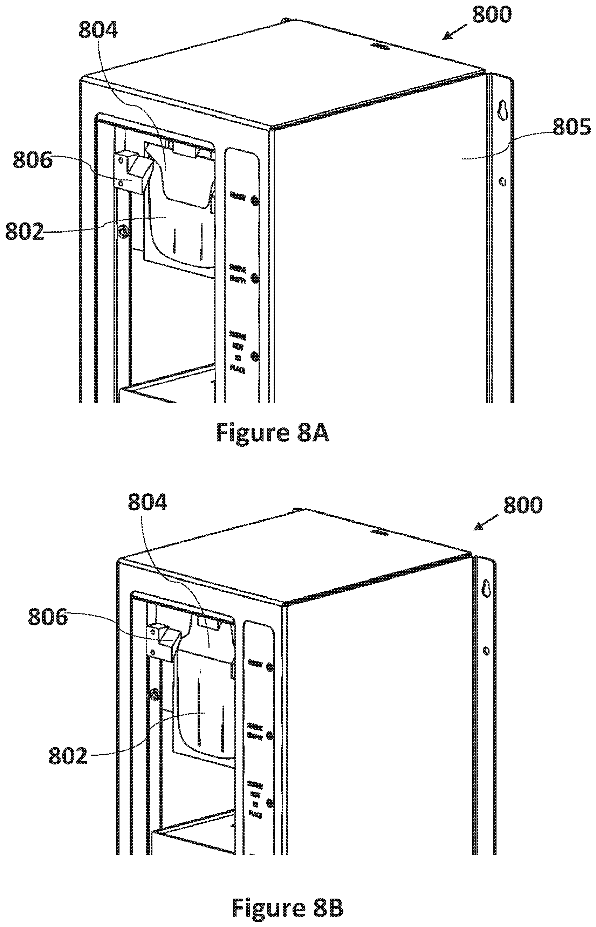

Referring now to Figs. 8A to 8B, which are simplified illustration of a perspective view of an envelope supply unit, according to some embodiments of the invention.

According to some embodiments, envelope supply unit **800** is configured to provide receptacles in the form of medication envelopes **802.** According to some embodiments, envelope **802** has a foldable flap **804.** As shown in Fig. 8A, flap **804** has a folded state, in which flap **804** is folded over the main body of envelope 802. In some embodiments, envelope **802** is stored in supply unit **800** in a folded state. In some embodiments, the folded state is a state in which envelope **802** is at its most compact size. As shown in Fig. 8B, flap **804** has an unfolded state, in which flap **804** is unfolded and is positioned away of the main body of envelope **802.** In some embodiments, the unfolded state is the state in which envelope **802** is shaped prior to transitioning envelope **802** to the receptacle carrier.

According to some embodiments, supply unit **800** has a supply unit housing **805** in which one or more envelopes **802** are stored. In some embodiments, unit **800** has one or more flap openers **806** coupled to housing **805** to open flap **804.** In to some embodiments, supply unit **800** is turning flap **804** of envelope **802** from a folded state to an unfolder state by moving envelope **802** downward, and pushing flap **804** by openers **806.** In some embodiments, feeding envelope **802** to an envelope carrier is by moving envelope **802** downwards and outside housing **805.** In some embodiments, feeding an envelope carrier is after coupling supply unit **800** to the carrier.

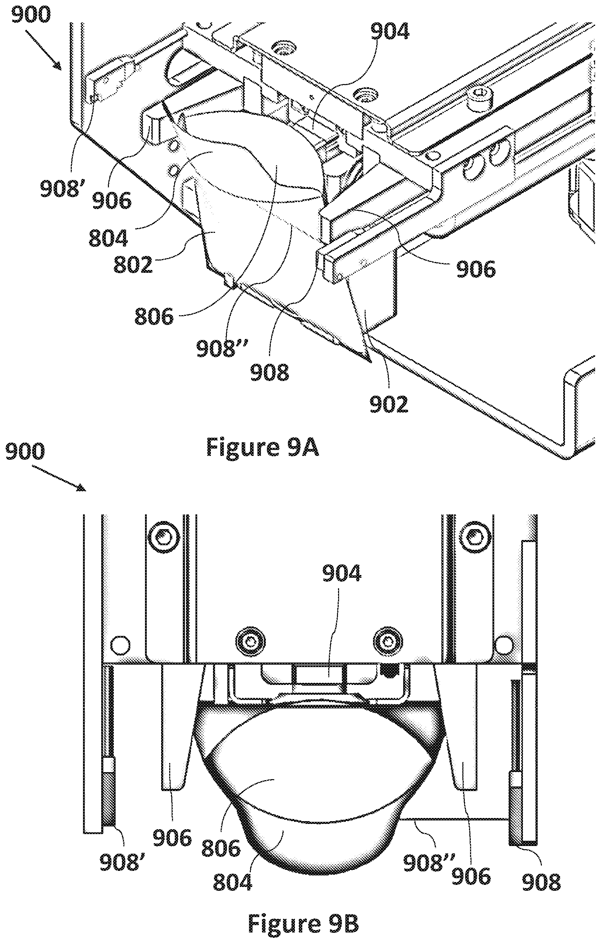

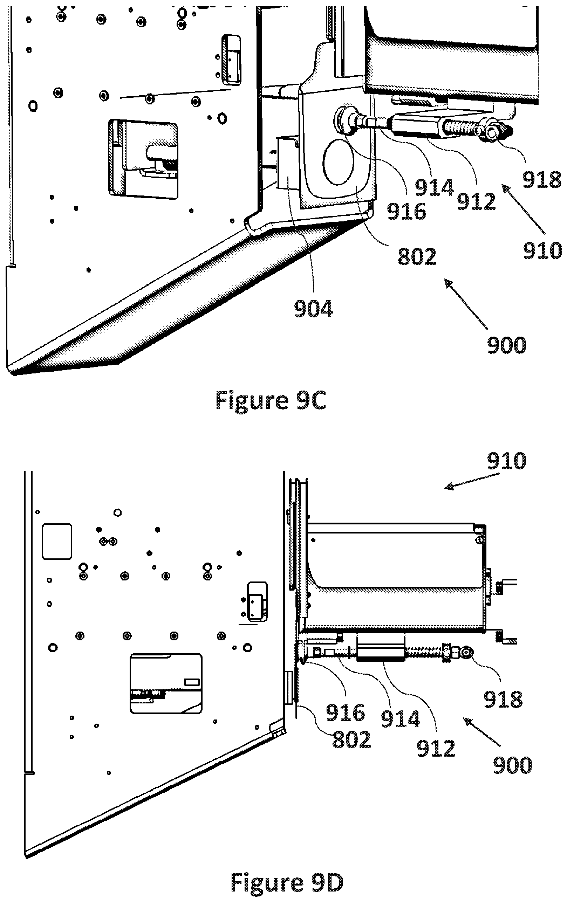

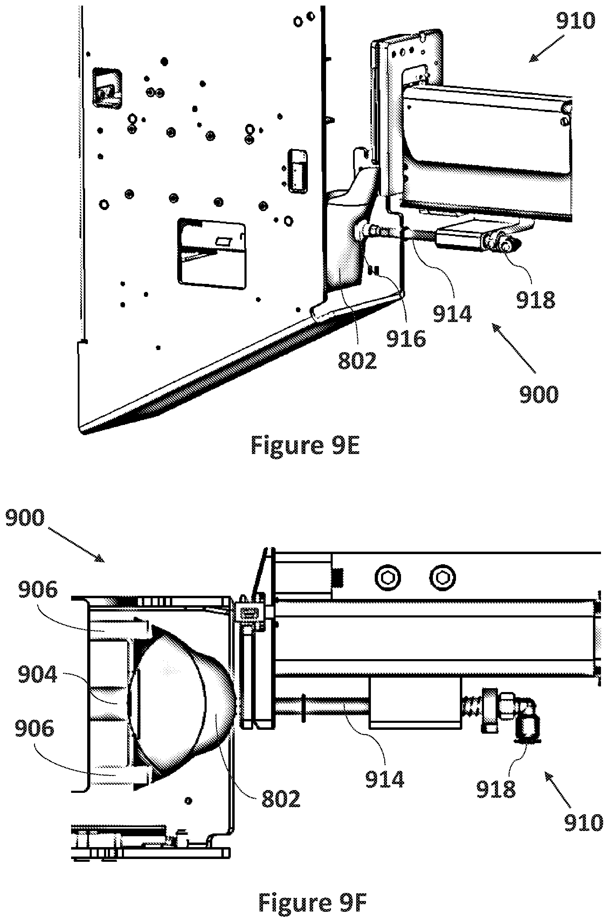

Referring now to Figs. 9A and 9B, which are simplified illustrations of a perspective view (9A) and a top view (9B) of a portion of an envelope carrier on which a medication envelope is mounted during the dispensing process, according to some embodiments of the invention.

Envelope carrier **900** can be one of the receptacle carriers described elsewhere herein (e.g. **618, 504).** As shown in Figs. 9A and 9B, envelope carrier **900** includes an envelope mount **902,** configured to hold envelope **802.** In some embodiments, mount **902** comprises one or more suction cups to couple envelope **802** to carrier **900** by applying suction on a face of envelope **802.** According to some embodiments, carrier **900** is configured to hold envelope **802** in an open state in which opening **806** is formed at its upper portion for receiving a medication dosage.

According to some embodiments, carrier includes retracting module **904** that retracts an upper portion of envelope **802** to form opening **806.** In some embodiments, retracting module **904** detracts an upper portion of envelope **802** to close opening **806.In** some embodiments, retracting module **904** is telescopic.

According to some embodiments, carrier **900** includes two forks **906** that hold envelope **802** in an open state. In some embodiments, forks **906** hold envelope **802,** while retracting module **904** retracts its upper portion. In some embodiments, forks **906** are movable to change the distance between them. In some embodiments, forks **906** clamp envelope **802** and reduce the distance between each other to open envelope **802.** In some embodiments, closing envelope **802** is by increasing the distance between forks **904.** In some embodiments, forks **906** are coupled to mount **902**.According to some embodiments, carrier **900** has an envelope sensor **908** to check if envelope **802** is in open state. In some embodiments, sensor **908** is configured to check the state of envelope **802** by transmitting an optical beam **908"** between a transmitter **908** and a receiver **908'.** In some embodiments, opening/closing of envelope **802** is initiated in accordance to signals of sensor **908.**

In some embodiments, carrier **900** has a flap sensor that checks if flap **804** is unfolded. In some embodiments, flap sensor is configured to check the state of flap **804** by sensing an interruption of an optical beam transmitted between a transmitter and receiver, in an unfolded state.

Referring now to Figs. 9C to 9F, which are simplified illustrations of perspective views (9C and 9E), a side view (9D), and a top view (9F) of a portion of an envelope carrier on which a medication envelope is mounted during the dispensing process, according to some embodiments of the invention.

As shown in Figs. 9C to 9F, an example embodiment of envelope carrier **900** comprises an envelope opener module **910** to modify the state of the envelope between a closed state (as shown in Figs. 9C-9D) and an open state (as shown in Figs. 9E-9F).

Envelope opener module **910,** comprises a bracket **912,** a manipulator **914** movably coupled to bracket **912** and having a proximal pulling head **916** and a distal end **918.**

In the example embodiment of Figs. 9C to 9F modifying the state of envelope **802** between closed state and open state is by engaging pulling head **916** with one face of envelope **802** and retracting module **904** with an opposite face. In some embodiments, opening is by coupling pulling head **916** and retracting module **904** to two opposite faces of envelope **802** and actuating one or more of pulling head **916** and retracting module **904** to move away from each other. Closing envelope **802** is by approximating pulling head **916** and retracting module **904.** In some embodiments, control circuitry initiates the moving of one or more of pulling head **916** and retracting module **904.**

According to some embodiments, coupling pulling head **916** with a face of envelope **802** is by suction. In some embodiments, suction is applied via distal end **918** through manipulator **914.** In some embodiments, coupling is by anchoring pulling head **916** to envelope **802.** In some embodiments, coupling is by applying a sticky material at pulling head **916.** In some embodiments, coupling is by applying an electrostatic force at pulling head **916.**

In some embodiments, manipulator **914** moves linearly towards and away of envelope **802** by a screwing within bracket **912.** In some embodiments, manipulator **914** is telescopic.

According to some embodiments, opener module **910** is coupled to envelope supply unit **800.** In some embodiments, opener module **910** and envelope supply unit **800** form a single unit. In some embodiments, carrier **900,** opener module **910,** and envelope supply unit **800** form a single unit. In some embodiments, dispensing head (as described elsewhere herein), carrier **900,** opener module **910,** and envelope supply unit **800** form a single unit.

### Other exemplary workflows of dispensing medication

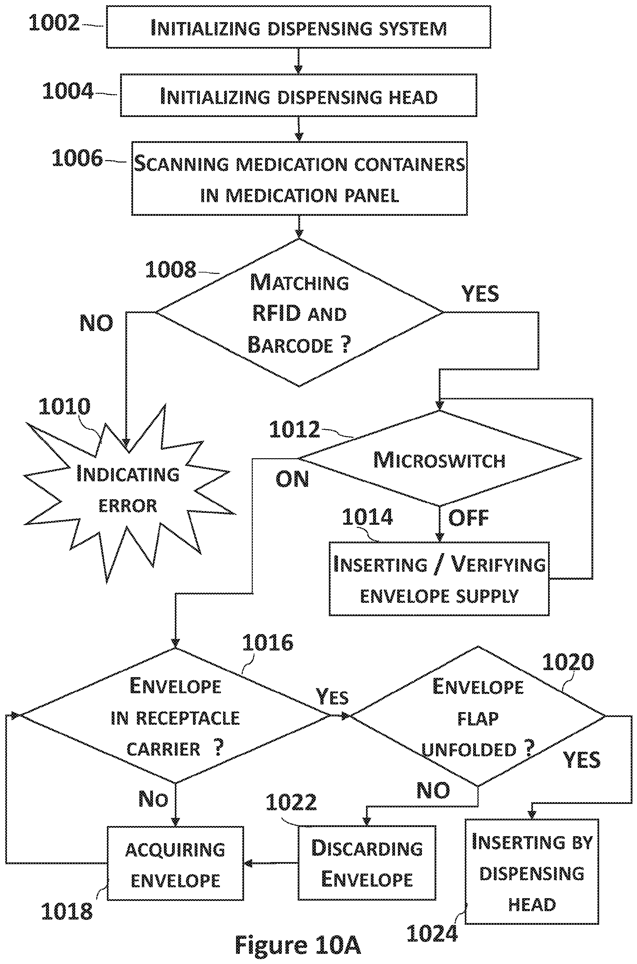

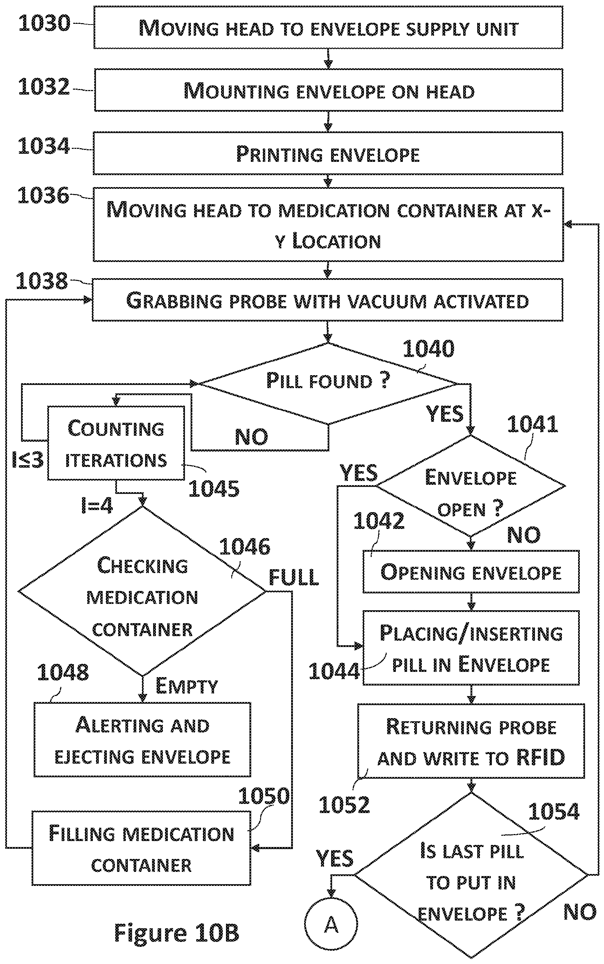

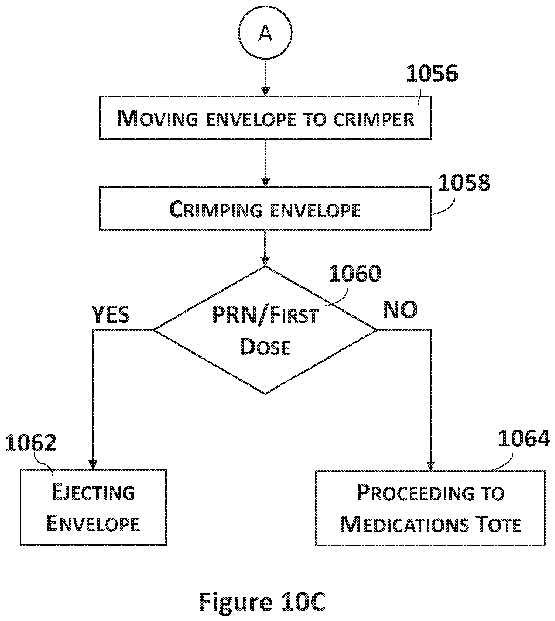

Referring now to Figs. 10A, 10B and 10C, which are simplified flow charts illustrating some of the activities related to operating a pharmaceutical dispensing system (such as 100), according to some embodiments of the invention.

As shown in Fig. 10A, the activities, according to some embodiments include:
**1002** Initializing the dispensing system.

In some embodiments, initializing **1002** comprises: preparing supply of medication. In some embodiments, initializing **1002** includes feeding system with prescriptions. In some embodiments, initializing **1002** includes Preparing supply of medication receptacle/envelopes. In some embodiments, initializing **1002** is for sensors, valves, etc. within the dispensing system. In some embodiments, initializing **1002** includes feeding control circuitry with movement plan. In some embodiments, initializing **1002** includes feeding control circuitry with time tables.
**1004** Initializing the dispensing head.
**1006** Scanning medication containers in medication panel.

Scanning can be for example for: evaluating correct supply of medication in containers, evaluating amount of medication available in medication panel, etc. In some embodiments, scanning is optional, and indication is provided by the dispensing system as described elsewhere herein.

In some embodiments, scanning is by the dispensing head. In some embodiments, scanning is by an operator.
**1008** Checking if RFID and/or barcode located at containers match values stored in a database.
**1010** Providing an indication about an error, if there is no match.

In some embodiments, correcting an error is by an operator/nurse. In some embodiments, correcting the error is by replacing a container. In some embodiments, replacing a container is done by accessing the medication container, and/or a cartridge attached to the medication container, from the medication panel. In some embodiment access to the container and/or cartridge does not require moving other containers. In some embodiment access to the container and/or cartridge does not require moving other containers using medications drawer.
**1012** checking if envelope supply is ready. In some embodiments, checking is by receiving a signal from a microswitch connected to an envelope storage box. In some embodiments, the signal is from sensors, such as: proximity sensor, pressure sensor, and weight sensor.
**1014** (optionally) if received an indication about envelopes not ready, verifying and/or inserting envelope supply in dispensing system.
**1016** (optionally) checking if envelope is in envelope carrier.

In some embodiments, this step is required when there is a need for acquiring another envelope, for example when there is an additional medication order in queue.
**1018** acquiring an envelope from envelope supply unit if checking **1016** indicates that there is no envelope in carrier.
**1020** (optionally) checking if envelope flap is unfolded.

According to some embodiments, the flap of the envelope is required to be unfolded in order to expand be body of the envelope for inserting medication within the envelope. In some embodiments, the flap of the envelope is required to be unfolded in order to print on the envelope.

According to some embodiments, checking for unfolded flap **1020** is by one or more sensors. In some embodiments, sensor is disposed at the dispensing head. In some embodiments, sensor is disposed at the envelope carrier. In some embodiments, checking **1020** if flap is unfolded is by a sensor sensing interruptions is light path. In some embodiments, checking **1020** if flap is unfolded is by pressure sensor receiving a pressure of the flap. In some embodiments, checking **1020** if flap is unfolded is by proximity sensor.
**1022** discarding envelope if checking **1020** revels the envelope flap is folded. In some embodiments, discarding **1022** is by the dispensing head, which discards the envelope to a discarding zone within the dispensing system. In some embodiments, discharging **1022** is to a discharging zone outside the dispensing system.
**1024** Inserting medication into the envelope.

As shown in Fig. 10B, according to some embodiments inserting **1024** includes:
**1030** Moving head to envelope supply unit.

According to some embodiments, envelope carrier is coupled to the dispensing head and moving **1030** includes moving of the envelope carrier.
**1032** Mounting envelope on head.

In some embodiments, moving **1030** and mounting **1032** are prior to checking **1016** if envelope is in envelope carrier. In some embodiments, moving **1030** and mounting **1032** are prior to checking **1020** (in Fig. 10A) if envelope flap is unfolded.
**1034** printing data on envelope.

In some embodiments, printing is on envelope having the flap in an unfolded state.
**1036** Moving dispensing head to proximate a medication container having medication required to be dispensed.

In some embodiments, moving **1036** includes outputting approximation signals by control circuitry to one or more actuators that move the dispending head to approximate the medication container.
**1038** Grabbing probe from medication container. In some embodiments, grabbing is by a probe gripping module coupled to the dispensing head. In some embodiments, a vacuum is activated by the dispensing head and applied on/via the probe.

In some embodiments, grabbing **1038** includes outputting manipulation signals by control circuitry to one or more actuators that move the dispending head or the gripping module.
**1040** checking if a pill is found/extracted by probe.

In some embodiments, a sensor disposed at the gripping module to check extracted pill. In some embodiments, checking is by measuring the value of the vacuum at the probe. In some embodiments, vacuum pressure value increases when a pill is picked by the probe.
**1041** checking if envelope is open.

In some embodiments, checking **1041** is by a sensor configured to measure the shape of the envelope to determine if it is open or flat.
**1042** opening envelope so it is ready for insertion of medication.

According to some embodiments, the envelope is in a flat (closed) state until opening **1042** by the dispensing system. In some embodiments, opening **1042** the envelope is by applying suction at a side of the envelope. In some embodiments, opening **1042** the envelope is by blowing air into the envelope by an air nozzle. In some embodiments, opening **1042** is by pressing two sides of the envelope.

Checking **1041** if envelope is open prior to extracting medication can potentially save loosing medication, by preventing dropping medication into a closed envelope. By checking **1041,** dispensing head is not actuated to dispense medication prior to having an envelope ready.

In some embodiments, the envelope is open after mounting **1032,** and steps **1041-1042** are optional. In some embodiments, opening **1042** is prior to mounting **1032.** In some embodiments, checking **1041** is prior to grabbing **1038** and potentially reduce loosing of medication, since medication is not extracted prior to having an envelope ready for dispensing. In some embodiments, checking **1041** is prior to moving **1036** and potentially reduce travel of the head towards medication, prior to having an open envelope.
**1044** placing/inserting pill in envelope.

In some embodiments, placing/inserting pill **1044** is by dropping the pill into envelope disposed under the pill after grabbing **1038.** In some embodiments, placing/inserting pill **1044** is by terminating a suction that holds the pill on a probe.
**1045** optionally repeat checking **1040** if pill is not found on probe. In some embodiments, repeating is for 2-10 times. In some embodiments, repeating is for 3-8 times. In some embodiments, repeating is for 4-6 times.
**1046** optionally checking of container has pills. In some embodiments, checking **1046** include counting the number of pills.

In some embodiments, when container is empty of pills, the dispensing system is configured to proceed to alerting and ejecting envelope **1048.** In some embodiments, when container is empty, the dispensing system is configured to locate an alternate location having the medication.

In some embodiments, alert is sent to another system in communication with the dispensing system. In some embodiments, an action depends on a response to the alert. In some embodiments, ejecting is by/to a user, which receives details about the alert. In some embodiments, envelope is marked to identify exception.
**1050** Filling container with medication.

According to some embodiments, the medication panel have redundancy of medications prepared in more than one medication assemblies. In some embodiments, redundancy reduced dispensing failures by moving the head, having an envelope, to another container without a critical alert, discarding the envelope, interruption, system idling, and/or requiring operator attention.

In some embodiments, after filling **1050,** the dispensing head proceed to repeat dispensing steps, by **1038** grabbing probe from medication container. In some embodiments, there is an optional step of closing envelope prior to repeating dispensing steps. In some embodiments, the envelope is closed between 80-98% of the time between moving **1030** and crimping **1058.** In some embodiments, the envelope is closed between 85-95% of the time between moving **1030** and crimping **1058.** In some embodiments, the envelope is closed between 90-93% of the time between moving **1030** and crimping **1058.** In some embodiments, envelope is open only between checking **1040** and placing pill **1044.** Closing the envelope between operations can potentially reduce contamination of medication disposed inside the envelope.

According to some embodiments, described elsewhere herein, the content of the containers assemblies is known prior to having the dispensing head approximating a container.
**1052** Returning probe and write to RFID.
**1054** Checking if inserted pill is the last pill required to be inserted in envelope.

If envelope should receive additional pills, the head will proceed to repeat from step **1036.**

If inserted the last pill required to be placed in envelope, the envelope can be moved **1056** (See Figure 10C) to a crimping module and crimping **1058.**
**1060** Checking PRN.
**1062** Ejecting envelope when checking PRN **1060** is positive.

According to some embodiments, checking PRN **1060** is positive when medication is required to be submit to the patient immediately (e.g. when patient is in pain or not yet in database or just enrolled in facility). In some embodiments, ejecting **1062** is to a nurse in a single manner and not through the medications tote.
**1064** Proceeding to position envelope in medication tote. In some embodiments, proceeding **1064** is on scheduled medication runs, so than checking PRN **1060** is negative.

One or more of the checking steps, mention above can be optional. The checking steps can potentially save redundant movements of the dispensing head, for example, when medication is not ready in container or envelope is not in a proper state, save extraction of medication when envelope is not ready, and save movement of head having a medication when envelope is not open.

### Extraction probe

According to some embodiments, a detachable probe **P** is coupled to the medication containers (such as **402)** for picking medication dosage. In some embodiments, extracting medication dosage is by grabbing probe **P** out of the medication container by the medication gripper (such as **106/608** described elsewhere herein).

According to some embodiments, the dispensing system has a control circuitry (such as **109)** that outputs manipulation signals to actuate the dispensing head to manipulate probe **P.** In some embodiments, the signals include velocity profile. In some embodiments, signals include acceleration profile. In some embodiments, signals include the length and the direction of movements. In some embodiments, the manipulation signals and/or the positioning signals are selected according to one or more parameters of the medication dosage. In some embodiment the control circuitry is coupled to the dispensing head.

In some embodiments, probe **P** has a tip configured to be disposed within the medication container, so that probe **P** can access medication disposed with the container. According to some embodiments, picking medication dosage is by applying suction through the tip of probe **P.** In some embodiments, suction is applied by the dispensing head and probe **P** is hollow to transfer suction to its tip. According to some embodiments, releasing the medication dosage of probe **P** is by terminating the suction.

In some embodiments, probe **P** is configured to pick medication by a grasping element disposed at the tip of the probe **P.** In some embodiments, grasping by probe **P** is without suction.

### Lost medication collector

Medication dosages may be lost/fall during a dispensing operation or due to other circumstances. According some embodiments, dispensing system **100** can be configured to for tracking and colleting medication dosage that failed to be disposed into receptacles **108.**

Turning back to Fig. 1, dispensing system **100** includes a lost medication collector **120** in which medication dosages, such as pills, can accumulate. In some embodiments, collector **120** is disposed at a bottom portion of panel **102.** In some embodiments, collector **120** is disposed below panel **102** (outside the panel). In some embodiments, collector **120** is removable. In some embodiments, collector **120** is in the form of a drawer. In some embodiments, collector **120** collects and conveys lost medication out dispensing system **100** automatically. In some embodiments, dispensing system **100** provides indications to the operator about one or more lost medication dosages.

A potential advantage of having a collector **120** is an increased usability of the dispensing system. Another potential advantage of having a collector **120** is increasing liability of the pharmacy by reducing the number of lost drugs and providing tracking records of the medications. Another potential advantage of having a collector **120** is reducing system downtime that could be required to release lost medication dosages.

In some embodiments, dispensing system **100** includes an internal camera **122.** In some embodiments, camera **122** can be used to locate medication dosage that failed to be disposed into receptacle **108.** In some embodiments, camera **122** is coupled to dispensing head **107.**

### Returning receptacles/envelopes

According to some embodiments, dispensing system **100** supports the process of returning receptacles/envelopes having medications. In some embodiments, closed receptacles/envelopes can be returned by storing returned receptacles/envelopes in a storage. In some embodiments, storage is configured to maintain the quality of the returned medication. In some embodiments, returning receptacles/envelopes includes scanning of the receptacles/envelopes by a scanner **128** connected or in communication with medication databases.

### Preparation steps

According to some embodiments, the dispensing process shown in Figs. 3A to 3D, includes some preparation activities performed by facility personnel, such as: system technician, nurse, and pharmacist. In some embodiments, the dispensing process is followed by activities on packaged medications performed by the facility personnel.

According to some embodiments, the preparations steps include feeding dispensing system (e.g. **100)** with prescription data. The prescription data can be of the patients in the healthcare facility.

According to some embodiments, the preparations steps include preparing a supply of medication receptacles (such as **108).**

According to some embodiments, the preparations steps include preparing the medication containers (such as **104).** In some embodiments, the medication containers comprise a cartridge by which medication is disposed within the container. In some embodiments, preparing is in accordance to data received about planned medication dosages. In some embodiments, the data is about medication types within prescription data. In some embodiments, preparing is in accordance to operational procedures unrelated to feeding of prescriptions.

According to some embodiments, the arrangement of medication containers **104** at panel **102** is optimized to reduce the travel trajectory of medication gripper **106.** In some embodiments, optimizing the arrangement is in accordance to historical data of the movements of medication gripper **106** and/or medication dispensed by system **100,** and/or a dispensing plan. In some embodiments, the location of medication containers **104** is determined by data about medication usage. In some embodiments, data is created by a computer **130** connected to the dispensing system 100. In some embodiments, data includes at least one of: prescribed medications, dispensed medication, medication extracted from medication containers **104.**

## Claims

1. A medication dispensing system, which inserts a probe to pick one single pill from one of one or more medication containers (402), and dispenses the one single pill into an opening in a medication receptacle, comprising:
a medication panel (400), having a plurality of docking ports (404) for accommodating said one or more medication containers (402);
one or more actuators;
a gripper (608) attachable to the probe or including the probe;
a receptacle carrier comprising a receptacle mount for holding the receptacle, and movable by the one or more actuators;
control circuitry, outputting positioning signals to the one or more actuators to move the receptacle carrier, and outputting dosage-manipulation signals to the one or more actuators to move the gripper (608) with the probe to allow the probe to pick and manipulate said pill out of the one of the one or more medicationcontainers (402); and
wherein a horizontal distance between the opening of the receptacle and said pill is less than 20 cm at least prior to outputting the dosage-manipulation signals.

2. The medication dispensing system according to claim 1, wherein said dispenses said pill into an opening in a medication receptacle comprises dropping said pill into said opening of said medication receptacle.

3. The medication dispensing system according to claim 1 or claim 2, wherein the positioning signals maintain a horizontal distance of less than 20 cm between a projection of the opening of the receptacle and a projection of the medication container on a horizontal plane.

4. The medication dispensing system according to any one of claims 1-3, wherein the dosage-manipulation signals manipulate said pill in a medication path, between the medication container and the opening of the medication receptacles, having a total horizontal length of less than 20 cm.

5. The medication dispensing system according to any one of claims 1-4, wherein the manipulation signals comprise rotating the gripper, between picking said pill out of the medication container and positioning said pill vertically above the opening of the receptacle.

6. The medication dispensing system according to any one of claims 1-5, wherein the one or more actuators move the receptacle carrier vertically between the medication containers.

7. The medication dispensing system according to any one of claims 1-6, wherein said receptacle is one or more medication envelopes, having an open state in which an upper side of the envelope is open for receiving said pill; and further comprising an envelope opener module having a manipulator, configured to open the envelope by coupling the manipulator to a face of the envelope.

8. The medication dispensing system according to claim 1, comprising a dispensing head, supporting the receptacle carrier to form a single unit that moves the receptacle together with the dispensing head; and . wherein the gripper is rotatably coupled to the dispensing head.

9. A method for dispensing medications in receptacles, using a dispensing system, having a gripper (608) for picking one single pill from one of one or more medication containers (402), and dispenses said into an opening in a medication receptacle, comprising:
extracting said single pill out of the one of one or more medication containers (402) by coupling the gripper (608) to a probe inserted in the medication container (402);
positioning a receptacle by a receptacle carrier in a horizontal distance of less than 20 cm between the medication dosage and the opening of the receptacle, at least prior to the extracting; and
dispensing said pill in the receptacle.

10. The method according to claim 9, comprising locating said pill to be vertically above the opening in the medication receptacle.

11. The method according to claim 9 or claim 10, wherein the positioning comprises one or more of:
a. moving the receptacle carrier in respect to the gripper;
b. is performed prior to the extracting;
c. maintaining an overlap between the projection of the opening of the receptacle and the projection of the medication container on a horizontal plane.

12. The method according to any one of claims 9-11, comprising one or more of:
a. opening the receptacle prior to the dispensing;
b. opening the receptacle between the positioning and the dispensing;
c. rotating the gripper to align with a probe inserted within the medication container prior to the extracting;
d. approximating the gripper to the medication container;

13. The method according to any one of claims 9-12, wherein the time between the extracting and the dispensing is less than 1 sec.

14. The method according to any one of claims 9-13, wherein the dispensing system comprises a dispensing head supporting the gripper, and the method comprises coupling the receptacle carrier and the dispensing head prior to the extracting.

15. The method according to any one of claims 9-14, wherein said dispensing said pill in the receptacle comprises dropping said pill into said opening of said medication receptacle.

## Patentansprüche

1. Arzneimittelabgabesystem, das eine Sonde einführt, um eine einzelne Tablette aus einem von einem oder mehreren Arzneimittelbehältnissen (402) aufzugreifen, und eine einzelne Tablette in eine Öffnung in einer Arzneimittelaufnahme abzugeben, umfassend:
eine Arzneimitteltafel (400), die eine Vielzahl von Docking-Ports (404) aufweist, in denen das eine oder die mehreren Arzneimittelbehältnisse (402) aufgenommen sind;
einen oder mehrere Aktuatoren;
einen Greifer (608), der an die Sonde angebracht werden kann oder die Sonde aufweist;
einen Aufnahmenträger, der eine Aufnahmenhalterung zum Halten der Aufnahme umfasst und durch den einen oder die mehreren Aktuatoren bewegt werden kann;
Steuerungsschalttechnik, die Positionierungssignale an den einen oder die mehreren Aktuatoren ausgibt, um den Aufnahmenträger zu bewegen, und Dosierungsmanipulationssignale an den einen oder die mehreren Aktuatoren ausgibt, um den Greifer (608) mit der Sonde zu bewegen, um der Sonde zu erlauben, die Tablette aus dem einen von dem einen oder den mehreren Arzneimittelbehältnissen (402) heraus zu greifen und zu manipulieren; und
wobei eine horizontale Distanz zwischen der Öffnung der Aufnahme und der Tablette, zumindest vor dem Ausgeben der Dosierungsmanipulationssignale, weniger als 20 cm beträgt.

2. Arzneimittelabgabesystem gemäß Anspruch 1, wobei das Abgeben der Tablette in eine Öffnung in einer Arzneimittelaufnahme das Fallenlassen der Tablette in die Öffnung der Arzneimittelaufnahme umfasst.

3. Arzneimittelabgabesystem gemäß Anspruch 1 oder Anspruch 2, wobei die Positionierungssignale eine horizontale Distanz von weniger als 20 cm zwischen einem Vorsprung der Öffnung der Aufnahme und einem Vorsprung des Arzneimittelbehältnisses auf einer horizontalen Ebene aufrechterhalten.

4. Arzneimittelabgabesystem gemäß einem der Ansprüche 1-3, wobei die Dosierungsmanipulationssignale die Tablette in einem Arzneimittelpfad zwischen dem Arzneimittelbehältnis und der Öffnung der Arzneimittelaufnahmen manipulieren, der eine horizontale Gesamtlänge von weniger als 20 cm aufweist.

5. Arzneimittelabgabesystem gemäß einem der Ansprüche 1-4, wobei die Manipulationssignale ein Drehen des Greifers zwischen dem Herausgreifen der Tablette aus dem Arzneimittelbehältnis und dem Positionieren der Tablette vertikal über der Öffnung der Aufnahme umfassen.

6. Arzneimittelabgabesystem gemäß einem der Ansprüche 1-5, wobei der eine oder die mehreren Aktuatoren den Aufnahmenträger vertikal zwischen den Arzneimittelbehältnissen bewegen.

7. Arzneimittelabgabesystem gemäß einem der Ansprüche 1-6, wobei die Aufnahme eine oder mehrere Arzneimittelhüllen ist, die einen offenen Zustand aufweisen, in dem eine obere Seite der Hülle zum Aufnehmen der Tablette offen ist;
und ferner umfassend ein Hüllenöffnermodul, das einen Manipulator aufweist, der dazu konfiguriert ist, die Hülle durch Koppeln des Manipulators mit einer Fläche der Hülle zu öffnen.

8. Arzneimittelabgabesystem gemäß Anspruch 1, umfassend einen Abgabekopf, der den Aufnahmenträger stützt, um eine einzelne Einheit zu bilden, die die Aufnahme zusammen mit dem Abgabekopf bewegt; und
wobei der Greifer mit dem Abgabekopf drehbar gekoppelt ist.

9. Verfahren zum Abgeben von Arzneimitteln in Aufnahmen, unter Verwendung eines Abgabesystems, das einen Greifer (608) zum Aufgreifen einer einzelnen Tablette aus einem von einem oder mehreren Arzneimittelbehältnissen (402) aufweist,
und Abgeben der Tablette in eine Öffnung in einer Arzneimittelaufnahme, umfassend:
Extrahieren der einzelnen Tablette aus dem einen von einem oder mehreren Arzneimittelbehältnissen (402) heraus durch Koppeln des Greifers (608) mit einer Sonde, die in das Arzneimittelbehältnis (402) eingeführt ist;
Positionieren einer Aufnahme durch einen Aufnahmenträger in einer horizontalen Distanz von weniger als 20 cm zwischen der Arzneimitteldosierung und der Öffnung der Aufnahme, zumindest vor dem Extrahieren; und
Abgeben der Tablette in die Aufnahme.

10. Verfahren gemäß Anspruch 9, umfassend das Platzieren der Tablette, um vertikal über der Öffnung in der Arzneimittelaufnahme zu sein.

11. Verfahren gemäß Anspruch 9 oder Anspruch 10, wobei das Positionieren eines oder mehrere von Folgendem umfasst:
a. Bewegen des Aufnahmenträgers in Bezug auf den Greifer;
b. wird vor dem Extrahieren durchgeführt;
c. Aufrechterhalten einer Überlappung zwischen dem Vorsprung der Öffnung der Aufnahme und dem Vorsprung des Arzneimittelbehältnisses auf einer horizontalen Ebene.

12. Verfahren gemäß einem der Ansprüche 9-11, das eines oder mehrere von Folgendem umfasst:
a. Öffnen der Aufnahme vor dem Abgeben;
b. Öffnen der Aufnahme zwischen dem Positionieren und dem Abgeben;
c. Drehen des Greifers zum Ausrichten auf eine Sonde, die vor dem Extrahieren in das Arzneimittelbehältnis eingeführt wird;
d. Annähern des Greifers an das Arzneimittelbehältnis.

13. Verfahren gemäß einem der Ansprüche 9-12, wobei die Zeit zwischen dem Extrahieren und dem Abgeben weniger als 1 Sek. beträgt.

14. Verfahren gemäß einem der Ansprüche 9-13, wobei das Abgabesystem einen Abgabekopf umfasst, der den Greifer stützt, und das Verfahren das Koppeln des Aufnahmenträgers und des Abgabekopfs vor dem Extrahieren umfasst.

15. Verfahren gemäß einem der Ansprüche 9-14, wobei das Abgeben der Tablette in die Aufnahme das Fallenlassen der Tablette in die Öffnung der Arzneimittelaufnahme umfasst.

## Revendications

1. Système de distribution de médicaments, qui insère une sonde pour prélever un seul comprimé depuis un d'un ou de plusieurs récipients de médicaments (402), et qui distribue un seul comprimé dans une ouverture d'un réceptacle de médicaments, comprenant :
un panneau de médicaments (400) présentant une pluralité d'orifices d'accueil (404) destinés à recevoir lesdits un ou plusieurs récipients de médicaments (402) ;
un ou plusieurs actionneurs :
un système de préhension (608) pouvant être fixé à la sonde ou comportant la sonde ;
un support de réceptacle comprenant un montant de réceptacle pour maintenir le réceptacle et pouvant être déplacé par les un ou plusieurs actionneurs ;
des circuits de commande, délivrant des signaux de positionnement aux un ou plusieurs actionneurs pour déplacer le support de réceptacle, et délivrant des signaux de manipulation de dose aux un ou plusieurs actionneurs pour déplacer le système de préhension (608) avec la sonde pour permettre à la sonde de prélever et de manipuler ledit comprimé hors du récipient de médicaments des un ou plusieurs récipients de médicaments (402) ; et
dans lequel une distance horizontale entre l'ouverture du réceptacle et ledit comprimé est inférieure à 20 cm au moins avant de délivrer les signaux de manipulation de dose.

2. Système de distribution de médicaments selon la revendication 1, dans lequel la distribution dudit comprimé dans une ouverture dans un réceptacle de médicaments comprend la chute dudit comprimé dans ladite ouverture du réceptacle de médicaments.

3. Système de distribution de médicaments selon la revendication 1 ou la revendication 2, dans lequel les signaux de positionnement maintiennent une distance horizontale inférieure à 20 cm entre une protubérance de l'ouverture du réceptacle et une protubérance du récipient de médicaments sur un plan horizontal.

4. Système de distribution de médicaments selon l'une quelconque des revendications 1-3, dans lequel les signaux de manipulation de dose manipulent ledit comprimé sur un trajet de médicament, entre le récipient de médicaments et l'ouverture des réceptacles de médicaments, présentant une longueur horizontale totale inférieure à 20 cm.

5. Système de distribution de médicaments selon l'une quelconque des revendications 1 - 4, dans lequel les signaux de manipulation comprennent la rotation du système de préhension, entre le prélèvement dudit comprimé hors du récipient de médicaments et le positionnement dudit comprimé verticalement au-dessus de l'ouverture du réceptacle.

6. Système de distribution de médicaments selon l'une quelconque des revendications 1-5, dans lequel les un ou plusieurs actionneurs déplacent le support de réceptacle de manière verticale entre les récipients de médicaments.

7. Système de distribution de médicaments selon l'une quelconque des revendications 1 - 6, dans lequel ledit réceptacle est une ou plusieurs enveloppes de médicament, présentant un état ouvert, dans lequel un côté supérieur de l'enveloppe est ouvert pour recevoir ledit comprimé ; et comprenant en outre un module ouvreur d'enveloppe présentant un système de manipulation configuré pour ouvrir l'enveloppe en couplant le système de manipulation à une face de l'enveloppe.

8. Système de distribution de médicaments selon la revendication 1, comprenant une tête de distribution, supportant le support de réceptacle pour former une unique unité qui déplace le réceptacle conjointement avec la tête de distribution ; et
dans lequel le système de préhension est couplé de manière à pouvoir tourner à la tête de distribution.

9. Procédé de distribution de médicaments dans des réceptacles en utilisant un système de distribution, présentant un système de préhension (608) destiné à prélever un seul comprimé d'un des un ou plusieurs récipients de médicaments (402), et ladite distribution dudit comprimé dans une ouverture dans un réceptacle de médicaments comprenant :
l'extraction dudit seul comprimé hors des un ou plusieurs récipients de médicaments (402) en couplant le système de préhension (608) à une sonde insérée dans le récipient de médicaments (402) ;
le positionnement d'un réceptacle par un support de réceptacle à une distance horizontale inférieure à 20 cm entre la dose de médicament et l'ouverture du réceptacle au moins avant l'extraction ; et
la distribution dudit comprimé dans le réceptacle.

10. Procédé selon la revendication 9, comprenant la localisation dudit comprimé de manière à être verticalement au-dessus de l'ouverture dans le réceptacle de médicaments.

11. Procédé selon la revendication 9 ou la revendication 10, dans lequel le positionnement comprend une ou plusieurs étapes :
a. de déplacement du support de réceptacle par rapport au système de préhension ;
b. qui est réalisé avant l'extraction ;
c. de maintien d'un chevauchement entre la protubérance de l'ouverture du réceptacle et la protubérance du récipient de médicaments sur un plan horizontal.

12. Procédé selon l'une quelconque des revendications 9 - 11, comprenant une ou plusieurs étapes :
a. d'ouverture du réceptacle avant la distribution ;
b. d'ouverture du réceptacle entre le positionnement et la distribution ;
c. de rotation du système de préhension pour l'alignement avec une sonde insérée dans le récipient de médicaments avant l'extraction ;
d. le rapprochement du système de préhension du récipient de médicaments.

13. Procédé selon l'une quelconque des revendications 9 - 12, dans lequel la durée entre l'extraction et la distribution est inférieure à 1 sec.

14. Procédé selon l'une quelconque des revendications 9 -13, dans lequel le système de distribution comprend une tête de distribution supportant le système de préhension, et le procédé comprend le couplage du support de réceptacle et la tête de distribution avant l'extraction.

15. Procédé selon l'une quelconque des revendications 9 - 14, dans lequel ladite distribution dudit comprimé dans le réceptacle comprend la chute dudit comprimé dans ladite ouverture dudit réceptacle de médicaments.
